# EUROPEAN PATENT SPECIFICATION

(11) **EP 2 112 184 B1**
(45) Date of publication and mention of the grant of the patent: **28.03.2012**
(21) Application number: 08704333.7
(22) Date of filing: 01.02.2008
(51) Int. Cl.: C08G 61/12, C08L 65/00, C09K 11/06, H01L 51/50

(54) **BLOCK COPOLYMER AND POLYMER LIGHT-EMITTING DEVICE**
BLOCKCOPOLYMER UND LICHTEMITTIERENDE POLYMERVORRICHTUNG
COPOLYMÈRE BLOC ET DISPOSITIF ÉLECTROLUMINESCENT À POLYMÈRE

(30) Priority: 01.02.2007 JP 2007022814
(43) Date of publication of application: 28.10.2009
(73) Proprietor: Sumitomo Chemical Company, Limited, Tokyo 104-8260 (JP)
(72) Inventor: NAKATANI, Tomoya, Tsukuba-shi Ibaraki 305-0821 (JP); KAKIMOTO, Hidenobu, Tsukuba-shi Ibaraki 305-0821 (JP)
(74) Representative: Duckworth, Timothy John
(86) International application number: PCT/JP2008/051624
(87) International publication number: WO 2008/093823

(56) References cited:
- WO-A1-2005/049546
- WO-A1-2006/037458
- WO-A2-2005/014688
- JP-A- 2004 534 863
- JP-A- 2005 515 264
- JP-A- 2007 162 009
- US-A1- 2003 045 642
- US-A1- 2005 038 223

## Description

### TECHNICAL FIELD

The present invention relates to a block copolymer and a polymer light-emitting device using the same.

### BACKGROUND ART

Since high-molecular-weight light-emitting materials and charge transport materials are useful as materials used for an organic layer in a light-emitting device, and the like, various types have been studied, and as an example, a polymer having a divalent group represented by the following formula as a repeating unit has been reported (for example, see PATENT DOCUMENT 1).

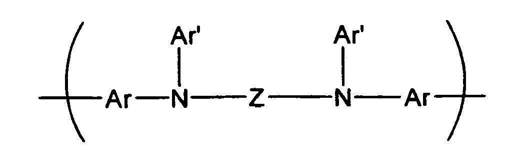

wherein Ar is a substituted or unsubstituted arylene group, Ar' is independently a substituted or unsubstituted aryl group, and Z is a polycyclic arylene group.
PATENT DOCUMENT 1: WO2005/049546

### DISCLOSURE OF THE INVENTION

### PROBLEMS TO BE SOLVED BY THE INVENTION

However, when the above copolymer is used as a light-emitting material for a light-emitting device, its emission luminance is not sufficient yet.
It is an object of the present invention to provide a block copolymer that can provide a light-emitting device that can provide high-luminance emission when it is used as a light-emitting material for a light-emitting device.

### MEANS FOR SOLVING THE PROBLEMS

The present invention provides a block copolymer characterized by comprising:
a block (A) comprising a repeating unit represented by the following formula (I), and a repeating unit represented by the following formula (II); and
a block (B) comprising a repeating unit represented by the following formula (II), a repeating unit represented by the following formula (III), and a repeating unit represented by the following formula (IV).

   -Ar₁- (I)

   wherein Ar₁ represents a divalent heterocyclic group containing a five-membered ring,

   -Ar₂- (II)

   wherein Ar₂ represents an arylene group,

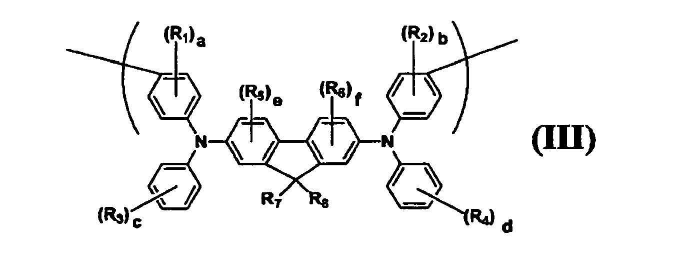

wherein R₁, R₂, R₃, R₄, R₅, and R₆ independently represent a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group,
a and b independently represent an integer of 0 to 4,
c and d independently represent an integer of 0 to 5, and
e and f independently represent an integer of 0 to 3,
provided that when a plurality of at least one of R₁, R₂, R₃, R₄, R₅, and R₆ are present, the plurality of atoms or groups may be the same or different,
R₇ and R₈ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and

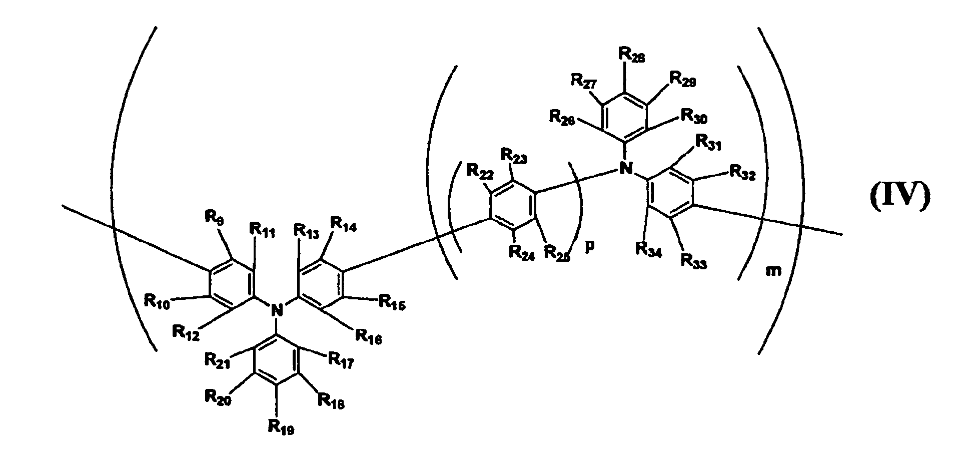

wherein R₉ to R₃₄ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and
m and p independently represent 0 or 1,
provided that in at least one of combinations of R₁₁ and R₁₃, R₁₂ and R₂₁, R₂₅ and R₃₄, and R₃₀ and R₃₁, the two groups may be directly bonded to each other to form a single bond or a ring as a divalent group.

Secondly, the present invention provides a composition characterized by comprising:
the block copolymer; and
a solvent, a light-emitting material other than the block copolymer, a hole transport material other than the block copolymer, or an electron transport material other than the block copolymer, or a combination of two or more thereof.

Thirdly, the present invention provides a light-emitting thin film characterized by comprising the block copolymer. Fourthly, the present invention provides a polymer light-emitting device characterized by comprising:
an anode;
a cathode; and
an organic layer comprising the block copolymer and provided between the anode and the cathode.

### ADVANTAGE OF THE INVENTION

The block copolymer of the present invention is useful as the light-emitting material, hole transport material, and electron transport material of a light-emitting device, and provides a light-emitting device that can provide high-luminance emission particularly when it is used as the light-emitting material of a light-emitting device. Therefore, a light-emitting device comprising the block copolymer of the present invention can be used for a curved and a planar light source for the backlight of a liquid crystal display or for illumination, a segment type display device, a dot matrix flat panel display, and the like.

### BEST MODE FOR CARRYING OUT THE INVENTION

### <Description of Terms>

Terms commonly used in this specification will be described below.

As a halogen atom, a fluorine atom, a chlorine atom, a bromine atom, and an iodine atom are illustrated.

The term "Cₓ to C_{y}" (x and y are positive integers satisfying x < y) represents that the number of the carbon atoms of an organic group described with this term is x to y.

An alkyl group means an unsubstituted alkyl group and an alkyl group substituted with a halogen atom or the like and includes both a linear alkyl group and a cyclic alkyl group (cycloalkyl group). The alkyl group may have a branch. The number of the carbon atoms of the alkyl group is usually about 1 to 20, preferably about 1 to 15, and more preferably about 1 to 10. The alkyl group includes, for example, a methyl group, an ethyl group, a propyl group, an isopropyl group, a butyl group, an isobutyl group, an s-butyl group, a t-butyl group, a pentyl group, an isoamyl group, a hexyl group, a cyclohexyl group, a heptyl group, an octyl group, a 2-ethylhexyl group, a nonyl group, a decyl group, a 3,7-dimethyloctyl group, a dodecyl group, a trifluoromethyl group, a pentafluoroethyl group, a perfluorobutyl group, a perfluorohexyl group, and a perfluorooctyl group. A C₁ to C₁₂ alkyl group includes, for example, a methyl group, an ethyl group, a propyl group, an isopropyl group, a butyl group, an isobutyl group, an s-butyl group, a t-butyl group, a pentyl group, an isoamyl group, a hexyl group, a cyclohexyl group, a heptyl group, an octyl group, a nonyl group, a decyl group, and a dodecyl group.

An alkoxy group means an unsubstituted alkoxy group and an alkoxy group substituted with a halogen atom, an alkoxy group, or the like, and includes both a linear alkoxy group and a cyclic alkoxy group (cycloalkoxy group). The alkoxy group may have a branch. The number of the carbon atoms of the alkoxy group is usually about 1 to 20, preferably about 1 to 15, and more preferably about 1 to 10. The alkoxy group includes, for example, a methoxy group, an ethoxy group, a propyloxy group, an isopropyloxy group, a butoxy group, an isobutoxy group, an s-butoxy group, a t-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, a dodecyloxy group, a trifluoromethoxy group, a pentafluoroethoxy group, a perfluorobutoxy group, a perfluorohexyloxy group, a perfluorooctyloxy group, a methoxymethyloxy group, and a 2-methoxyethyloxy group. A C₁ to C₁₂ alkoxy group includes, for example, a methoxy group, an ethoxy group, a propyloxy group, an isopropyloxy group, a butoxy group, an isobutoxy group, an s-butoxy group, a t-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, and a dodecyloxy group.

An alkylthio group means an unsubstituted alkylthio group and an alkylthio group substituted with a halogen atom or the like and includes both a linear alkylthio group and a cyclic alkylthio group (cycloalkylthio group). The alkylthio group may have a branch. The number of the carbon atoms of the alkylthio group is usually about 1 to 20, preferably about 1 to 15, and more preferably about 1 to 10. The alkylthio group includes, for example, a methylthio group, an ethylthio group, a propylthio group, an isopropylthio group, a butylthio group, an isobutylthio group, an s-butylthio group, a t-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, a dodecylthio group, and a trifluoromethylthio group. A C₁ to C₁₂ alkylthio group includes, for example, a methylthio group, an ethylthio group, a propylthio group, an isopropylthio group, a butylthio group, an isobutylthio group, an s-butylthio group, a t-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, and a dodecylthio group.

An aryl group is a remaining atomic group obtained by removing from an aromatic hydrocarbon one hydrogen atom bonded to a carbon atom constituting an aromatic ring and means an unsubstituted aryl group and an aryl group substituted with a halogen atom, an alkoxy group, an alkyl group, or the like. The aryl group also includes one having a condensed ring, and one in which two or more independent benzene rings or condensed rings are bonded via a single bond or a divalent organic group, for example, an alkenylene group, such as a vinylene group. The number of the carbon atoms of the aryl group is usually about 6 to 60, preferably about 7 to 48, and more preferably about 7 to 30. The aryl group includes, for example, a phenyl group, a C₁ to C₁₂ alkoxyphenyl group, a C₁ to C₁₂ alkylphenyl group, a 1-naphthyl group, a 2-naphthyl group, a 1-anthracenyl group, a 2-anthracenyl group, a 9-anthracenyl group, and a pentafluorophenyl group. Among them, a C₁ to C₁₂ alkoxyphenyl group and a C₁ to C₁₂ alkylphenyl group are preferred.

The C₁ to C₁₂ alkoxyphenyl group includes, for example, a methoxyphenyl group, an ethoxyphenyl group, a propyloxyphenyl group, an isopropyloxyphenyl group, a butyloxyphenyl group, an isobutyloxyphenyl group, a t-butyloxyphenyl group, a pentyloxyphenyl group, a hexyloxyphenyl group, and an octyloxyphenyl group.

The C₁ to C₁₂ alkylphenyl group includes, for example, a methylphenyl group, an ethylphenyl group, a dimethylphenyl group, a propylphenyl group, a mesityl group, a methylethylphenyl group, an isopropylphenyl group, a butylphenyl group, an isobutylphenyl group, a t-butylphenyl group, a pentylphenyl group, an isoamylphenyl group, a hexylphenyl group, a heptylphenyl group, an octylphenyl group, a nonylphenyl group, a decylphenyl group, and a dodecylphenyl group.

An aryloxy group means an unsubstituted aryloxy group and an aryloxy group substituted with a halogen atom, an alkoxy group, an alkyl group, or the like. The number of the carbon atoms of the aryloxy group is usually about 6 to 60, preferably about 7 to 48, and more preferably about 7 to 30. The aryloxy group includes, for example, a phenoxy group, a C₁ to C₁₂ alkoxyphenoxy group, a C₁ to C₁₂ alkylphenoxy group, a 1-naphthyloxy group, a 2-naphthyloxy group, and a pentafluorophenyloxy group. Among them, a C₁ to C₁₂ alkoxyphenoxy group and a C₁ to C₁₂ alkylphenoxy group are preferred.

The C₁ to C₁₂ alkoxyphenoxy group includes, for example, a methoxyphenoxy group, an ethoxyphenoxy group, a propyloxyphenoxy group, an isopropyloxyphenoxy group, a butyloxyphenoxy group, an isobutyloxyphenoxy group, a t-butyloxyphenoxy group, a pentyloxyphenoxy group, a hexyloxyphenoxy group, and an octyloxyphenoxy group.

The C₁ to C₁₂ alkylphenoxy group includes, for example, a methylphenoxy group, an ethylphenoxy group, a dimethylphenoxy group, a propylphenoxy group, a 1,3,5-trimethylphenoxy group, a methylethylphenoxy group, an isopropylphenoxy group, a butylphenoxy group, an isobutylphenoxy group, an s-butylphenoxy group, a t-butylphenoxy group, a pentylphenoxy group, an isoamylphenoxy group, a hexylphenoxy group, a heptylphenoxy group, an octylphenoxy group, a nonylphenoxy group, a decylphenoxy group, and a dodecylphenoxy group.

An arylthio group means an unsubstituted arylthio group and an arylthio group substituted with a halogen atom, an alkoxy group, an alkyl group, or the like. The number of the carbon atoms of the arylthio group is usually about 6 to 60, preferably about 7 to 48, and more preferably about 7 to 30. The arylthio group includes, for example, a phenylthio group, a C₁ to C₁₂ alkoxyphenylthio group, a C₁ to C₁₂ alkylphenylthio group, a 1-naphthylthio group, a 2-naphthylthio group, and a pentafluorophenylthio group.

An arylalkyl group means an unsubstituted arylalkyl group and an arylalkyl group substituted with a halogen atom, an alkoxy group, an alkyl group, or the like. The number of the carbon atoms of the arylalkyl group is usually about 7 to 60, preferably about 7 to 48, and more preferably about 7 to 30. The arylalkyl group includes, for example, a phenyl-C₁ to C₁₂ alkyl group, a C₁ to C₁₂ alkoxyphenyl-C₁ to C₁₂ alkyl group, a C₁ to C₁₂ alkylphenyl-C₁ to C₁₂ alkyl group, a 1-naphthyl-C₁ to C₁₂ alkyl group, and a 2-naphthyl-C₁ to C₁₂ alkyl group.

An arylalkoxy group means an unsubstituted arylalkoxy group and an arylalkoxy group substituted with a halogen atom, an alkoxy group, an alkyl group, or the like. The number of the carbon atoms of the arylalkoxy group is usually about 7 to 60, preferably about 7 to 48, and more preferably about 7 to 30. The arylalkoxy group includes, for example, a phenyl-C₁ to C₁₂ alkoxy group, a C₁ to C₁₂ alkoxyphenyl-C₁ to C₁₂ alkoxy group, a C₁ to C₁₂ alkylphenyl-C₁ to C₁₂ alkoxy group, a 1-naphthyl-C₁ to C₁₂ alkoxy group, and a 2-naphthyl-C₁ to C₁₂ alkoxy group.

An arylalkylthio group means an unsubstituted arylalkylthio group and an arylalkylthio group substituted with a halogen atom, an alkoxy group, an alkyl group, or the like. The number of the carbon atoms of the arylalkylthio group is usually about 7 to 60, preferably about 7 to 48, and more preferably about 7 to 30. The arylalkylthio group includes, for example, a phenyl-C₁ to C₁₂ alkylthio group, a C₁ to C₁₂ alkoxyphenyl-C₁ to C₁₂ alkylthio group, a C₁ to C₁₂ alkylphenyl-C₁ to C₁₂ alkylthio group, a 1-naphthyl-C₁ to C₁₂ alkylthio group, and a 2-naphthyl-C₁ to C₁₂ alkylthio group.

An arylalkenyl group means an unsubstituted arylalkenyl group and an arylalkenyl group substituted with a halogen atom, an alkoxy group, an alkyl group, or the like. The number of the carbon atoms of the arylalkenyl group is usually about 8 to 60, preferably about 8 to 48, and more preferably about 8 to 30. The arylalkenyl group includes, for example, a phenyl-C₂ to C₁₂ alkenyl group, a C₁ to C₁₂ alkoxyphenyl-C₂ to C₁₂ alkenyl group, a C₁ to C₁₂ alkylphenyl-C₂ to C₁₂ alkenyl group, a 1-naphthyl-C₂ to C₁₂ alkenyl group, and a 2-naphthyl-C₂ to C₁₂ alkenyl group. Among them, a C₁ to C₁₂ alkoxyphenyl-C₂ to C₁₂ alkenyl group and a C₁ to C₁₂ alkylphenyl-C₂ to C₁₂ alkenyl group are preferred.

The C₂ to C₁₂ alkenyl group includes, for example, a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group.

An arylalkynyl group means an unsubstituted arylalkynyl group and an arylalkynyl group substituted with a halogen atom, an alkoxy group, an alkyl group, or the like. The number of the carbon atoms of the arylalkynyl group is usually about 8 to 60, preferably about 8 to 48, and more preferably about 8 to 30. The arylalkynyl group includes, for example, a phenyl-C₂ to C₁₂ alkynyl group, a C₁ to C₁₂ alkoxyphenyl-C₂ to C₁₂ alkynyl group, a C₁ to C₁₂ alkylphenyl-C₂ to C₁₂ alkynyl group, a 1-naphthyl-C₂ to C₁₂ alkynyl group, and a 2-naphthyl-C₂ to C₁₂ alkynyl group. Among them, a C₁ to C₁₂ alkoxyphenyl-C₂ to C₁₂ alkynyl group and a C₁ to C₁₂ alkylphenyl-C₂ to C₁₂ alkynyl group are preferred.

The C₂ to C₁₂ alkynyl group includes, for example, an ethynyl group, a 1-propynyl group, a 2-propynyl group, a 1-butynyl group, a 2-butynyl group, a 1-pentynyl group, a 2-pentynyl group, a 1-hexynyl group, a 2-hexynyl group, and a 1-octynyl group.

A monovalent heterocyclic group refers to a remaining atomic group obtained by removing one hydrogen atom from a heterocyclic compound and means an unsubstituted monovalent heterocyclic group and a monovalent heterocyclic group substituted with a substituent, such as an alkyl group. The number of the carbon atoms of the monovalent heterocyclic group is usually about 4 to 60, preferably about 4 to 30, and more preferably about 4 to 20, not including the number of the carbon atoms of the substituent. Here, the heterocyclic compound refers to, among organic compounds having a cyclic structure, one containing, as elements constituting a ring, not only a carbon atom, but also a heteroatom, such as an oxygen atom, a sulfur atom, a nitrogen atom, a phosphorus atom, a boron atom, a silicon atom, a selenium atom, a tellurium atom, and an arsenic atom. The monovalent heterocyclic group includes, for example, a thienyl group, a C₁ to C₁₂ alkylthienyl group, a pyrrolyl group, a furyl group, a pyridyl group, a C₁ to C₁₂ alkylpyridyl group, a pyridazinyl group, a pyrimidyl group, a pyrazinyl group, a triazinyl group, a pyrrolidyl group, a piperidyl group, a quinolyl group, and an isoquinolyl group. Among them, a thienyl group, a C₁ to C₁₂ alkylthienyl group, a pyridyl group, and a C₁ to C₁₂ alkylpyridyl group are preferred.

A heterocyclic thio group means a group in which the hydrogen atom of a mercapto group is substituted with a monovalent heterocyclic group. The heterocyclic thio group includes, for example, a heteroarylthio group, such as a pyridylthio group, a pyridazinylthio group, a pyrimidylthio group, a pyrazinylthio group, and a triazinylthio group.

An amino group means an unsubstituted amino group and an amino group substituted with one or two substituents selected from an alkyl group, an aryl group, an arylalkyl group, and a monovalent heterocyclic group (hereinafter referred to as a substituted amino group). The substituent may further have a substituent (hereinafter sometimes referred to as a secondary substituent). The number of the carbon atoms of the substituted amino group is usually about 1 to 60, preferably about 2 to 48, and more preferably about 2 to 40, not including the number of the carbon atoms of the secondary substituent. The substituted amino group includes, for example, a methylamino group, a dimethylamino group, an ethylamino group, a diethylamino group, a propylamino group, a dipropylamino group, an isopropylamino group, a diisopropylamino group, a butylamino group, an isobutylamino group, an s-butylamino group, a t-butylamino group, a pentylamino group, a hexylamino group, a heptylamino group, an octylamino group, a 2-ethylhexylamino group, a nonylamino group, a decylamino group, a 3,7-dimethyloctylamino group, a dodecylamino group, a cyclopentylamino group, a dicyclopentylamino group, a cyclohexylamino group, a dicyclohexylamino group, a ditrifluoromethylamino group, a phenylamino group, a diphenylamino group, a C₁ to C₁₂ alkoxyphenylamino group, a di (C₁ to C₁₂ alkoxyphenyl)amino group, a C₁ to C₁₂ alkylphenylamino group, a di (C₁ to C₁₂ alkylphenyl)amino group, a 1-naphthylamino group, a 2-naphthylamino group, a pentafluorophenylamino group, a pyridylamino group, a pyridazinylamino group, a pyrimidylamino group, a pyrazinylamino group, a triazinylamino group, a phenyl-C₁ to C₁₂ alkylamino group, a C₁ to C₁₂ alkoxyphenyl-C₁ to C₁₂ alkylamino group, a di (C₁ to C₁₂ alkoxyphenyl-C₁ to C₁₂ alkyl)amino group, a C₁ to C₁₂ alkylphenyl-C₁ to C₁₂ alkylamino group, a di (C₁ to C₁₂ alkylphenyl-C₁ to C₁₂ alkyl)amino group, a 1-naphthyl-C₁ to C₁₂ alkylamino group, and a 2-naphthyl-C₁ to C₁₂ alkylamino group.

A silyl group means an unsubstituted silyl group and a silyl group substituted with one, two, or three substituents selected from an alkyl group, an aryl group, an arylalkyl group, and a monovalent heterocyclic group (hereinafter referred to as a substituted silyl group). The substituent may have a secondary substituent. The number of the carbon atoms of the substituted silyl group is usually about 1 to 60, preferably about 3 to 48, and more preferably about 3 to 40, not including the number of the carbon atoms of the secondary substituent. The substituted silyl group includes, for example, a trimethylsilyl group, a triethylsilyl group, a tripropylsilyl group, a triisopropylsilyl group, a dimethyl-isopropylsilyl group, a diethyl-isopropylsilyl group, a t-butylsilyldimethylsilyl group, a pentyldimethylsilyl group, a hexyldimethylsilyl group, a heptyldimethylsilyl group, an octyldimethylsilyl group, a 2-ethylhexyl-dimethylsilyl group, a nonyldimethylsilyl group, a decyldimethylsilyl group, a 3,7-dimethyloctyl-dimethylsilyl group, a dodecyldimethylsilyl group, a phenyl-C₁ to C₁₂ alkylsilyl group, a C₁ to C₁₂ alkoxyphenyl-C₁ to C₁₂ alkylsilyl group, a C₁ to C₁₂ alkylphenyl-C₁ to C₁₂ alkylsilyl group, a 1-naphthyl-C₁ to C₁₂ alkylsilyl group, a 2-naphthyl-C₁ to C₁₂ alkylsilyl group, a phenyl-C₁ to C₁₂ alkyldimethylsilyl group, a triphenylsilyl group, a tri-p-xylylsilyl group, a tribenzylsilyl group, a diphenylmethylsilyl group, a t-butyldiphenylsilyl group, and a dimethylphenylsilyl group.

An acyl group means an unsubstituted acyl group and an acyl group substituted with a halogen atom or the like. The number of the carbon atoms of the acyl group is usually about 2 to 20, preferably about 2 to 18, and more preferably about 2 to 16. The acyl group includes, for example, an acetyl group, a propionyl group, a butyryl group, an isobutyryl group, a pivaloyl group, a benzoyl group, a trifluoroacetyl group, and a pentafluorobenzoyl group.

An acyloxy group means an unsubstituted acyloxy group and an acyloxy group substituted with a halogen atom or the like. The number of the carbon atoms of the acyloxy group is usually about 2 to 20, preferably about 2 to 18, and more preferably about 2 to 16. The acyloxy group includes, for example, an acetoxy group, a propionyloxy group, a butyryloxy group, an isobutyryloxy group, a pivaloyloxy group, a benzoyloxy group, a trifluoroacetyloxy group, and a pentafluorobenzoyloxy group.

An imine residue means a residue obtained by removing, from an imine compound having a structure represented by at least one of the formula: H-N=C< and the formula: -N=CH-, one hydrogen atom in this structure. Such an imine compound includes, for example, aldimine, ketimine, and a compound in which the hydrogen atom bonded to the nitrogen atom in aldimine is substituted with an alkyl group, an aryl group, an arylalkyl group, an arylalkenyl group, an arylalkynyl group, or the like. The number of the carbon atoms of the imine residue is usually about 2 to 20, preferably about 2 to 18, and more preferably about 2 to 16. The imine residue includes, for example, a group represented by the general formula: -CR''=N-R''' or the general formula: -N=C(R''')₂ wherein R'' represents a hydrogen atom, an alkyl group, an aryl group, an arylalkyl group, an arylalkenyl group, or an arylalkynyl group, and R"' independently represents an alkyl group, an aryl group, an arylalkyl group, an arylalkenyl group, or an arylalkynyl group, provided that when two R'''s are present, the two "'s may be bonded to each other and taken together to form a ring as a divalent group, for example, an alkylene group having 2 to 18 carbon atoms, such as an ethylene group, a trimethylene group, a tetramethylene group, a pentamethylene group, and a hexamethylene group. Specific examples of the imine residue include groups represented by the following structural formulas:

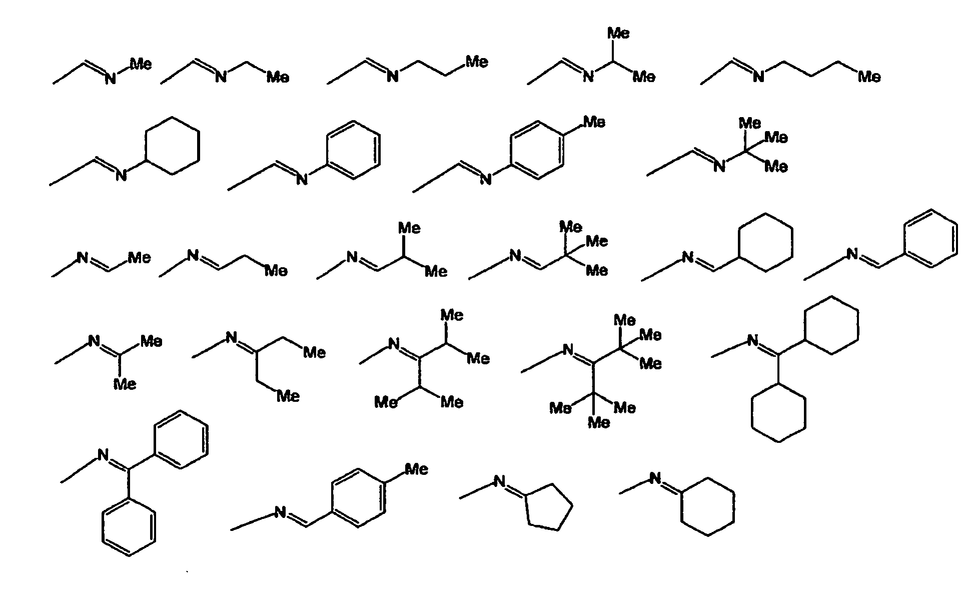

wherein Me represents a methyl group.

An amide group means an unsubstituted amide group and an amide group substituted with a halogen atom or the like. The number of the carbon atoms of the amide group is usually about 2 to 20, preferably about 2 to 18, and more preferably about 2 to 16. As the amide group, for example, a formamide group, an acetamide group, a propioamide group, a butyroamide group, a benzamide group, a trifluoroacetamide group, a pentafluorobenzamide group, a diformamide group, a diacetamide group, a dipropioamide group, a dibutyroamide group, a dibenzamide group, a ditrifluoroacetamide group, and a dipentafluorobenzamide group are illustrated.

An acid imide group means a residue obtained by removing the hydrogen atom bonded to the nitrogen atom from acid imide. The number of the carbon atoms of the acid imide group is usually about 4 to 20, preferably about 4 to 18, and more preferably about 4 to 16. The acid imide group includes, for example, groups shown below:

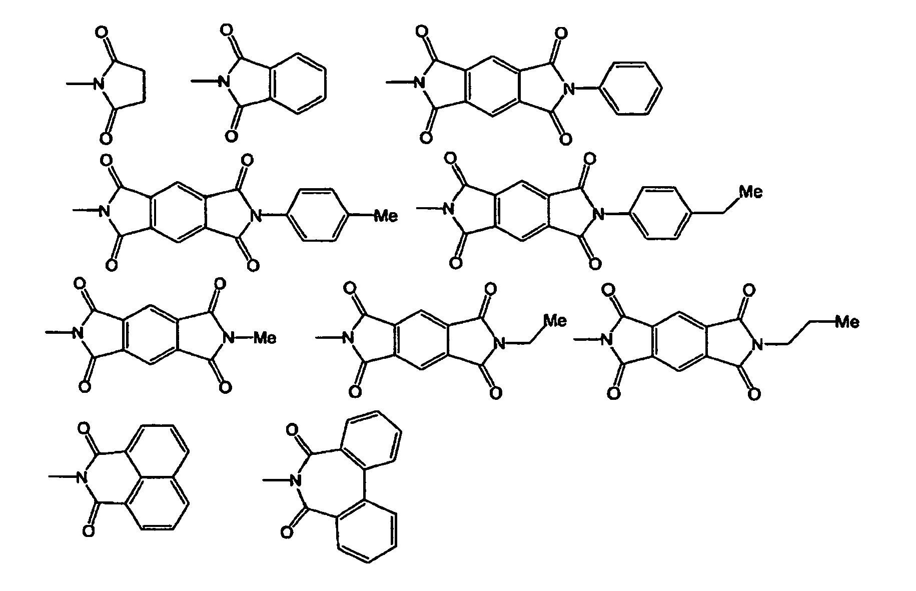

wherein Me represents a methyl group.

A carboxyl group means an unsubstituted carboxyl group and a carboxyl group substituted with a substituent, such as an alkyl group, an aryl group, an arylalkyl group, and a monovalent heterocyclic group (hereinafter referred to as a substituted carboxyl group). The substituent may have a secondary substituent. The number of the carbon atoms of the substituted carboxyl group is usually about 1 to 60, preferably about 2 to 48, and more preferably about 2 to 45, not including the number of the carbon atoms of the secondary substituent. The substituted carboxyl group includes, for example, a methoxycarbonyl group, an ethoxycarbonyl group, a propoxycarbonyl group, an isopropoxycarbonyl group, a butoxycarbonyl group, an isobutoxycarbonyl group, an s-butoxycarbonyl group, a t-butoxycarbonyl group, a pentyloxycarbonyl group, a hexyloxycarbonyl group, a cyclohexyloxycarbonyl group, a heptyloxycarbonyl group, an octyloxycarbonyl group, a 2-ethylhexyloxycarbonyl group, a nonyloxycarbonyl group, a decyloxycarbonyl group, a 3,7-dimethyloctyloxycarbonyl group, a dodecyloxycarbonyl group, a trifluoromethoxycarbonyl group, a pentafluoroethoxycarbonyl group, a perfluorobutoxycarbonyl group, a perfluorohexyloxycarbonyl group, a perfluorooctyloxycarbonyl group, a phenoxycarbonyl group, a naphthoxycarbonyl group, and a pyridyloxycarbonyl group.

### <Repeating Unit Represented by Formula (I)>

The block copolymer of the present invention comprises a repeating unit represented by the following formula (I) in the block (A). The block copolymer of the present invention may comprise two or more different types of repeating units represented by the following formula (I) in the block (A).

-Ar₁- (I)

wherein Ar₁ represents a divalent heterocyclic group containing a five-membered ring.

The divalent heterocyclic group containing a five-membered ring refers to a remaining atomic group obtained by removing two hydrogen atoms from a heterocyclic compound containing a five-membered ring and means an unsubstituted divalent heterocyclic group containing a five-membered ring, and a substituted divalent heterocyclic group containing a five-membered ring. Here, the heterocyclic compound containing a five-membered ring refers to, among organic compounds having a cyclic structure containing a five-membered ring, one containing, as elements constituting a ring, not only a carbon atom, but also a heteroatom, such as an oxygen atom, a sulfur atom, a nitrogen atom, a phosphorus atom, a boron atom, a silicon atom, a selenium atom, a tellurium atom, and an arsenic atom. Among the divalent heterocyclic groups containing a five-membered ring, a divalent aromatic heterocyclic group containing a five-membered ring is preferred.
The substituted atom and substituent in the substituted divalent heterocyclic group containing a five-membered ring are not particularly limited. However, from the viewpoint of solubility, fluorescence characteristics, the ease of synthesis, characteristics when a device is formed, and the like, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group is preferred. The number of the carbon atoms of a portion excluding the substituent in the divalent heterocyclic group containing a five-membered ring is usually about 3 to 60, preferably about 3 to 50, and more preferably about 3 to 40. Also, the total number of the carbon atoms of the divalent heterocyclic group containing a five-membered ring, including the substituent, is usually about 3 to 100, preferably about 3 to 80, and more preferably about 3 to 70.

The divalent heterocyclic group containing a five-membered ring includes, for example, the following:
- groups containing, as a heteroatom, an oxygen atom, a silicon atom, a nitrogen atom, a sulfur atom, a selenium atom, or the like and having a fluorene structure (the following formulas 141 to 155),
- five-membered ring-containing heterocyclic groups containing, as a heteroatom, an oxygen atom, a silicon atom, a nitrogen atom, a sulfur atom, a selenium atom, a boron atom, a phosphorus atom, or the like (the following formulas 156 to 175),
- five-membered ring-containing condensed heterocyclic groups containing, as a heteroatom, an oxygen atom, a silicon atom, a nitrogen atom, a sulfur atom, a selenium atom, or the like (the following formulas 176 to 187),

- five-membered ring-containing heterocyclic groups containing, as a heteroatom, an oxygen atom, a silicon atom, a nitrogen atom, a sulfur atom, a selenium atom, or the like, in which heterocycles are bonded to each other at the α -position of the heteroatom to form a dimer or an oligomer (the following formulas 188 to 189),
- five-membered ring-containing heterocyclic groups containing, as a heteroatom, an oxygen atom, a silicon atom, a nitrogen atom, a sulfur atom, a selenium atom, or the like, in which a heterocycle and a phenylene group are bonded to each other at the α-position of the heteroatom (the following formulas 190 to 196), and
- groups in which a five-membered ring-containing condensed heterocycle containing, as a heteroatom, an oxygen atom, a nitrogen atom, a sulfur atom, a selenium atom, or the like is substituted by a phenylene group, a furandiyl group, or a thiophenediyl group (the following formulas 197 to 202).

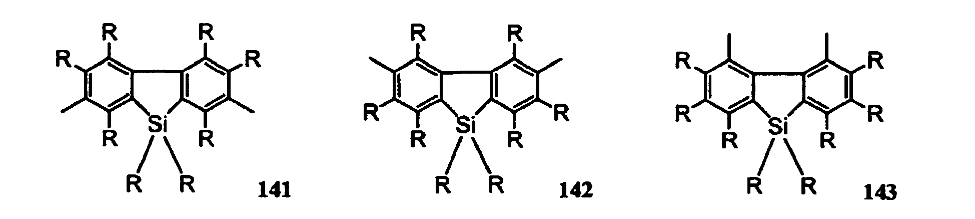

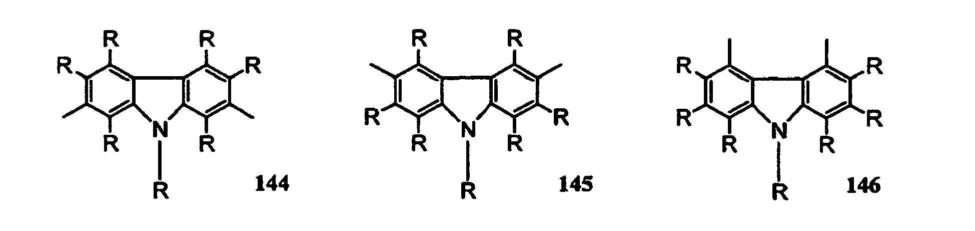

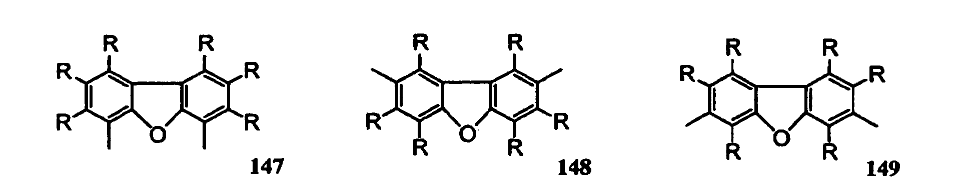

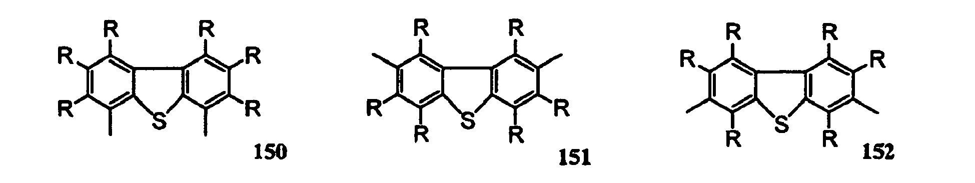

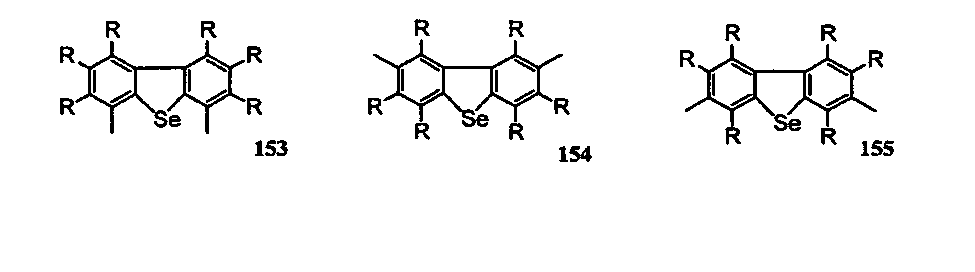

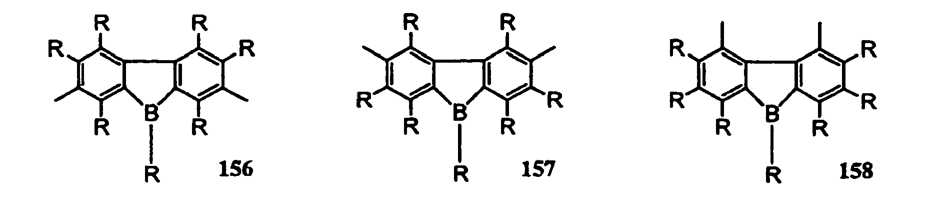

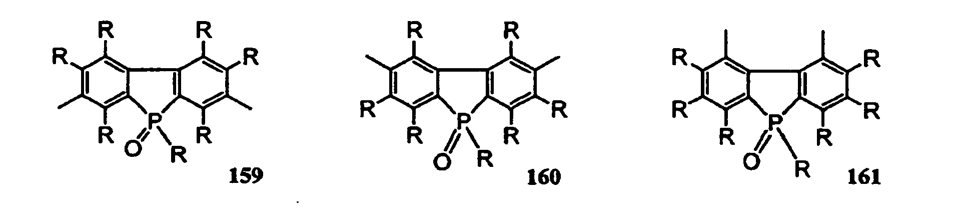

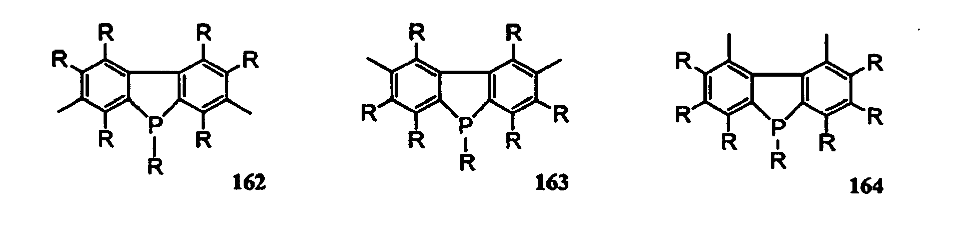

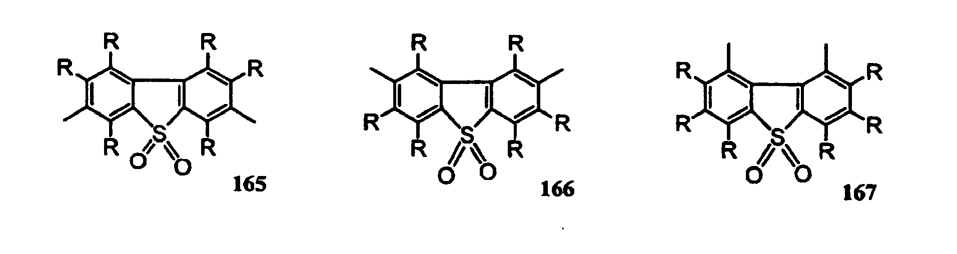

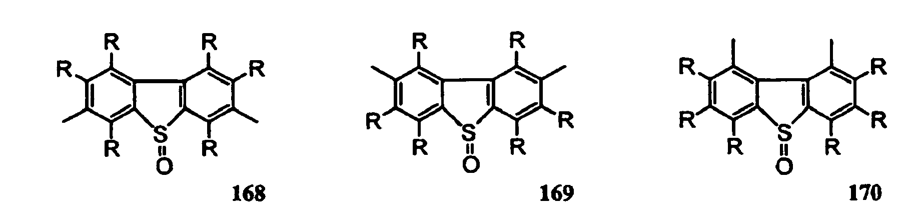

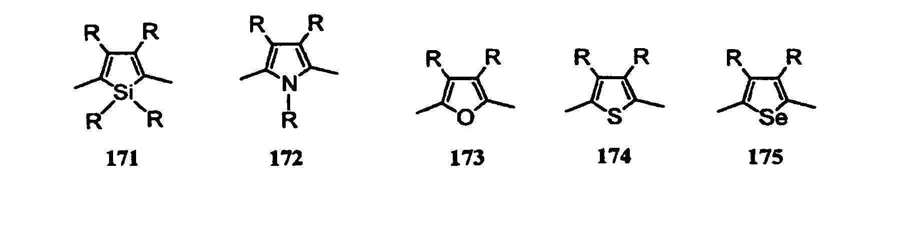

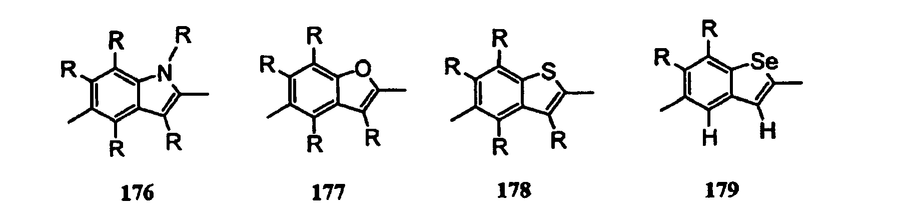

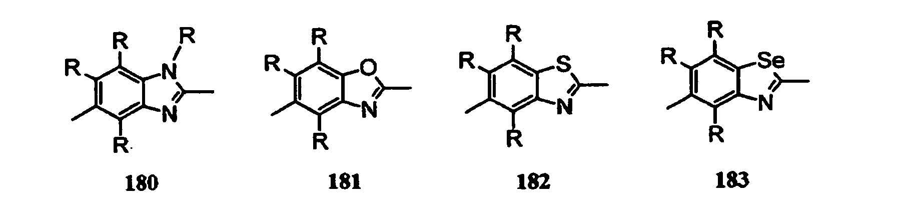

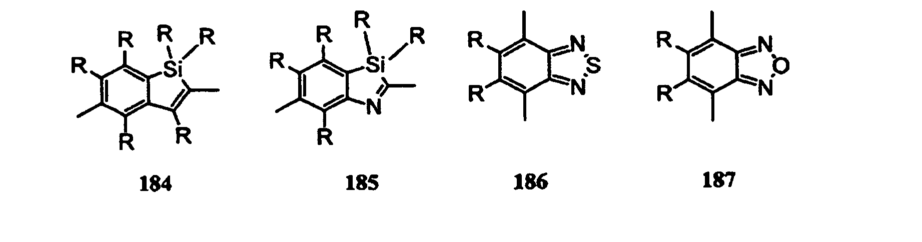

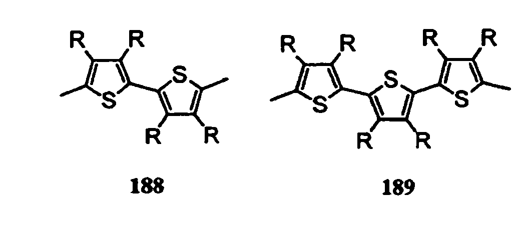

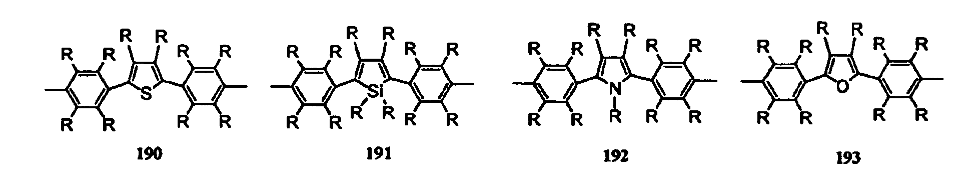

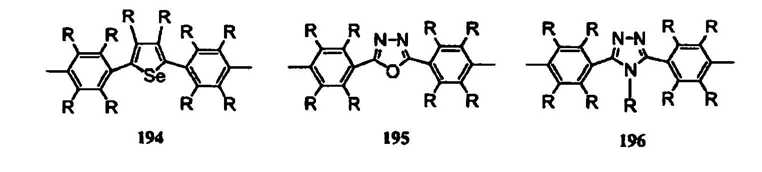

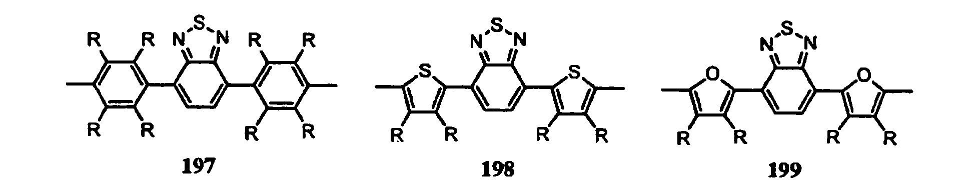

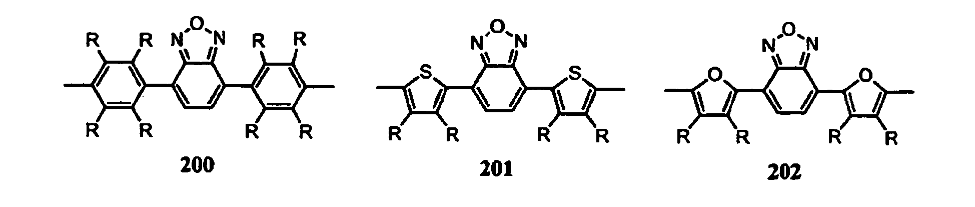

In the above formulas 141 to 202, R represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group.

From the viewpoint of providing a device having high luminous efficiency, in the above formula (I), Ar₁ is preferably a group represented by the following formula (V):

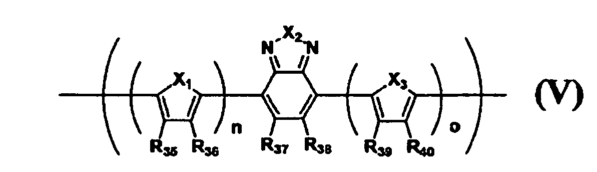

wherein R₃₅, R₃₆, R₃₇, R₃₈, R₃₉, and R₄₀ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group,
n and o independently represent an integer of 0 to 3, and
X₁, X₂, and X₃ independently represent an oxygen atom or a sulfur atom,
provided that when a plurality of at least one of R₃₅, R₃₆, R₃₉, R₄₀, X₁, and X₃ are present, the plurality of atoms or groups may be the same or different.

From the viewpoint of the ease of the synthesis of the raw material monomer, in the above formula (V), R₃₅, R₃₆, R₃₇, R₃₈, R₃₉, and R₄₀ are preferably independently a hydrogen atom, an alkyl group, an alkoxy group, an alkylthio group, an aryl group, an aryloxy group, an arylthio group, or a heterocyclic thio group, more preferably a hydrogen atom, an alkyl group, an alkoxy group, an aryl group, or an aryloxy group, and further preferably a hydrogen atom, an alkyl group, or an aryl group.

From the viewpoint of the ease of the synthesis of the raw material monomer, in the above formula (V), n and o are preferably independently an integer of 0 to 2, and further preferably 0 or 1.

Further, from the viewpoint of the durability of the block copolymer of the present invention, in the above formula (V), X₁, X₂, and X₃ are preferably an oxygen atom or a sulfur atom, and preferably a sulfur atom.

Specific examples of the repeating unit represented by the above formula (V) include repeating units represented by the following formulas (V-1) to (V-9):

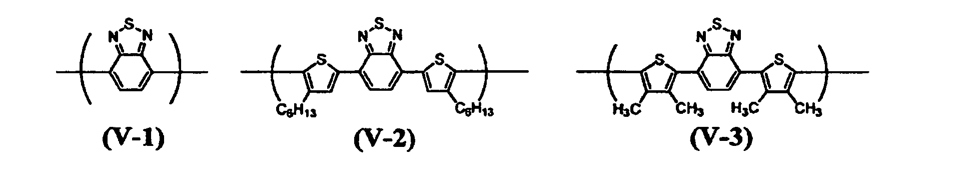

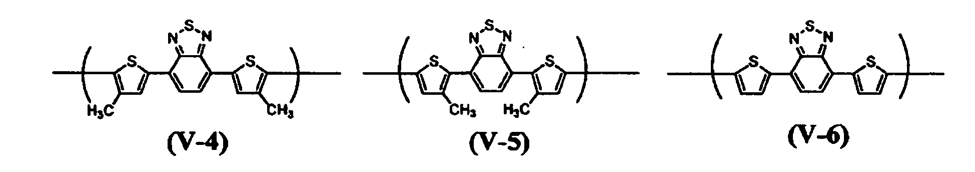

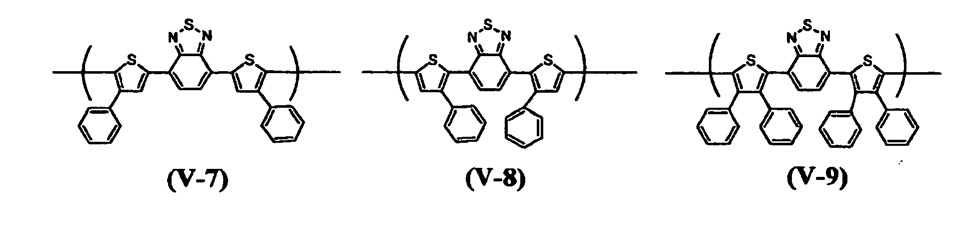

### <Repeating Unit Represented by Formula (II)>

The block copolymer of the present invention has a repeating unit represented by the following formula (II), in addition to the repeating unit represented by the above formula (I), in the block (A). Also, the block copolymer of the present invention has the repeating unit represented by the following formula (II) in the block (B). The block copolymer of the present invention may have two or more different types of repeating units represented by the following formula (II) in either of the blocks (A) and (B).

-Ar₂- (II)

wherein Ar₂ represents an arylene group.

The arylene group is a remaining atomic group obtained by removing from an aromatic hydrocarbon two hydrogen atoms bonded to carbon atoms constituting an aromatic ring and means an unsubstituted arylene group and a substituted arylene group. The arylene group also includes one having a condensed ring, and one in which two or more independent benzene rings or condensed rings are bonded via a single bond or a divalent organic group, for example, an alkenylene group, such as a vinylene group. The substituted atom and substituent in the substituted arylene group are not particularly limited. However, from the viewpoint of solubility, fluorescence characteristics, the ease of synthesis, characteristics as a material of a device, and the like, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group is preferred.

The number of the carbon atoms of a portion excluding the substituent in the arylene group is usually about 6 to 60, preferably about 6 to 20, and more preferably about 6 to 18. Also, the total number of the carbon atoms of the arylene group including the substituent is usually about 6 to 100, preferably about 6 to 80, and more preferably about 6 to 70.

The arylene group includes, for example, a phenylene group (for example, the following formulas 1 to 3), a naphthalenediyl group (for example, the following formulas 4 to 13), an anthracene-diyl group (for example, the following formulas 14 to 19), a biphenyl-diyl group (for example, the following formulas 20 to 25), a terphenyl-diyl group (for example, the following formulas 26 to 28), a fluorenediyl group (for example, the following formulas 36 to 38), a benzofluorene-diyl group (for example, the following formulas 39 to 46), and other divalent condensed polycyclic aromatic hydrocarbon groups (for example, the following formulas 29 to 35).

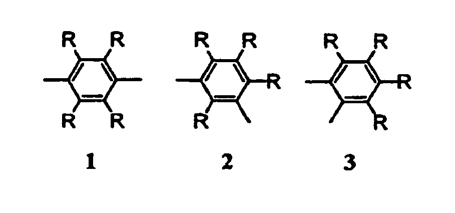

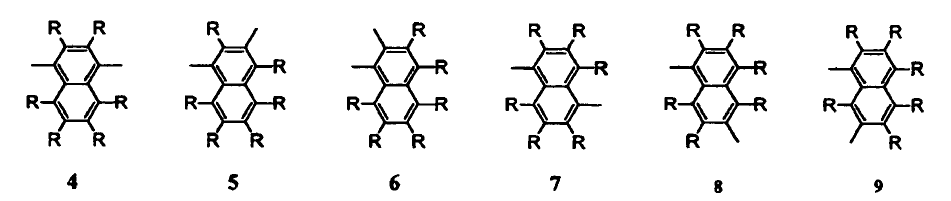

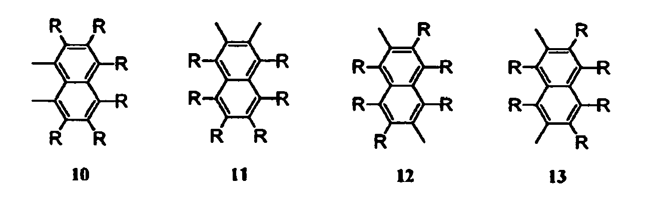

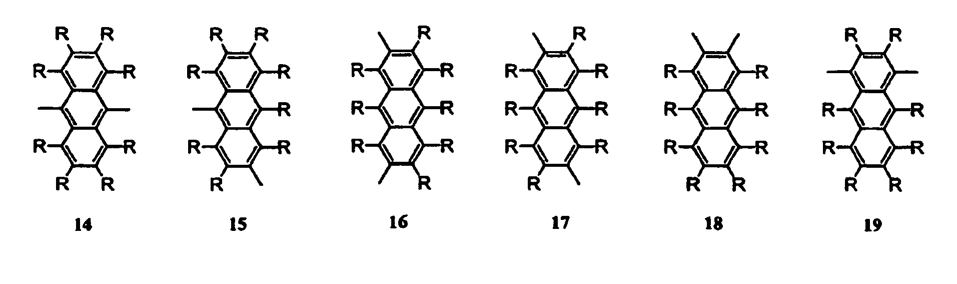

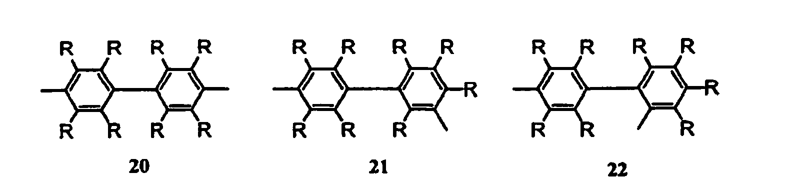

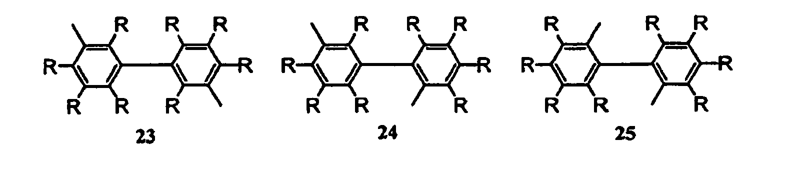

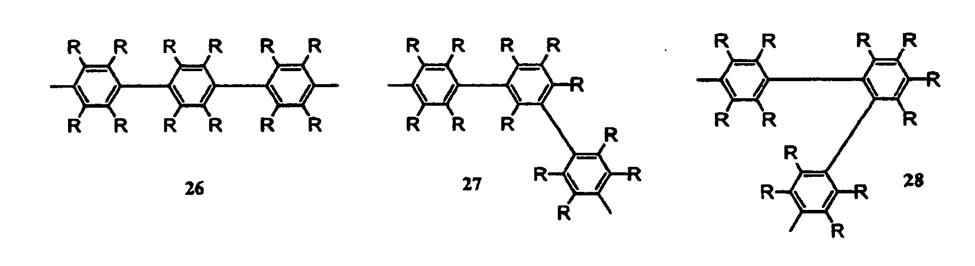

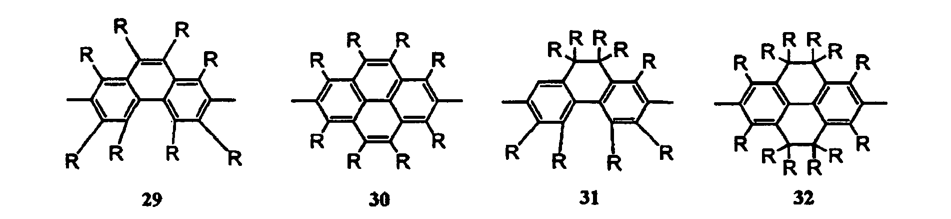

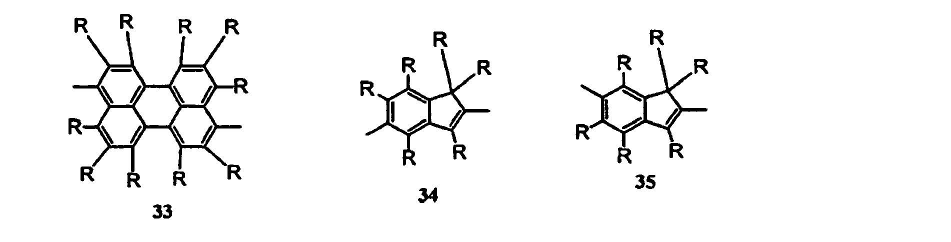

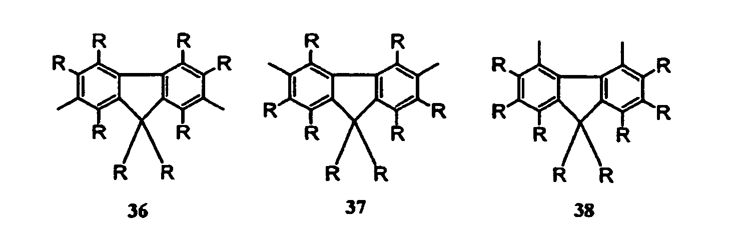

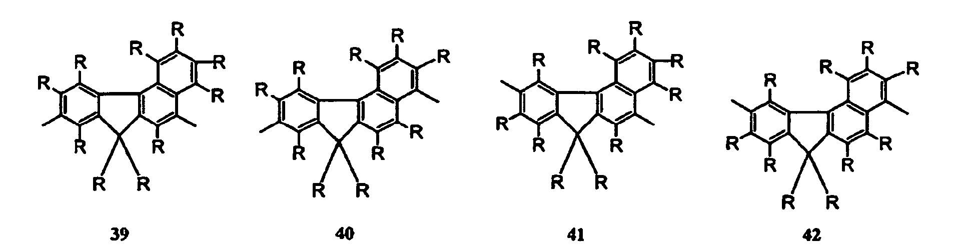

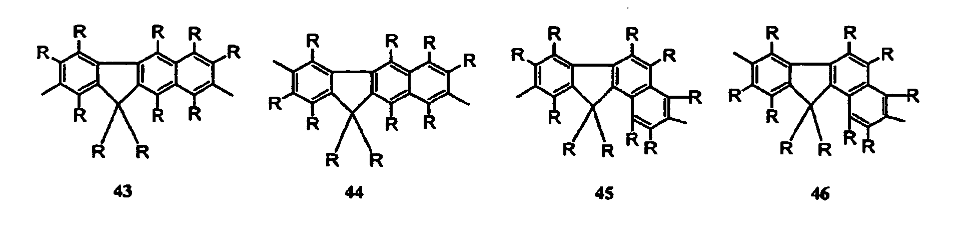

In the above formulas 1 to 46, R represents the same meaning as the above.

From the viewpoint of the durability of a light-emitting device using the block copolymer of the present invention, in the above formula (II), Ar₂ is preferably a group represented by the following formula (VI):

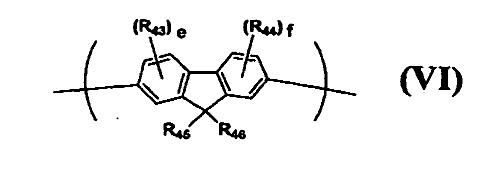

wherein R₄₃ and R₄₄ independently represent a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and
e and f independently represent an integer of 0 to 3,
provided that when a plurality of at least one of R₄₃ and R₄₄ are present, the plurality of atoms or groups may be the same or different, and
R₄₅ and R₄₆ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group.

From the viewpoint of the ease of the synthesis of the raw material monomer, in the above formula (VI), R₄₃ and R₄₄ are preferably an alkyl group or an alkoxy group.

From the viewpoint of the ease of the synthesis of the raw material monomer, in the above formula (VI), e and f are preferably 0 or 1, and most preferably 0.

From the viewpoint of the ease of the synthesis of the raw material monomer, in the above formula (VI), R₄₅ and R₄₆ are preferably an alkyl group or an aryl group.

Specific examples of the group represented by the above formula (VI) include groups represented by the following formulas (VI-1) to (VI-8):

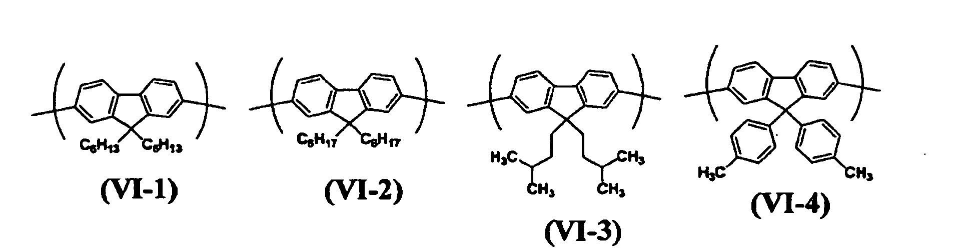

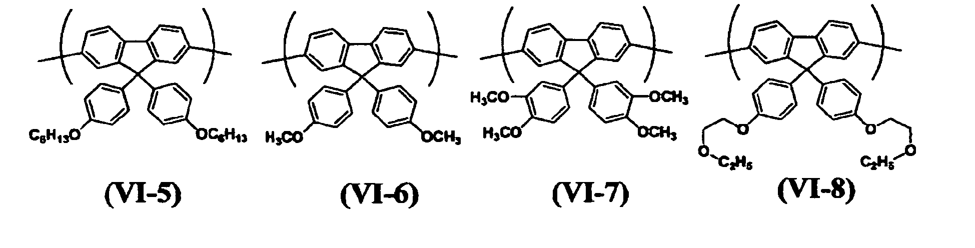

### <Repeating Unit Represented by Formula (III)>

The block copolymer of the present invention has a repeating unit represented by the following formula (III), in addition to the repeating unit represented by the above formula (II), in the block (B). The block copolymer of the present invention may have two or more different types of repeating units represented by the following formula (III) in the block (B).

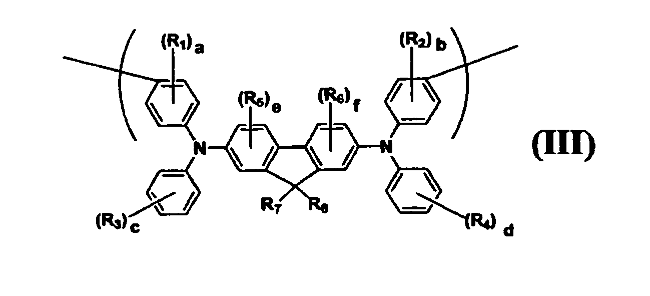

wherein R₁, R₂, R₃, R₄, R₅, and R₆ independently represent a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group,
a and b independently represent an integer of 0 to 4,
c and d independently represent an integer of 0 to 5, and
e and f independently represent an integer of 0 to 3,
provided that when a plurality of at least one of R₁, R₂, R₃, R₄, R₅, and R₆ are present, the plurality of atoms or groups may be the same or different, and
R₇ and R₈ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group.

From the viewpoint of the ease of the synthesis of the raw material monomer, in the above formula (III), a, b, e, and f are preferably 0 or 1, and further preferably 0.

From the viewpoint of the ease of the synthesis of the raw material monomer, in the above formula (III), c and d are preferably an integer of 0 to 3, and further preferably an integer of 1 to 3.

From the viewpoint of the ease of the synthesis of the raw material monomer, in the above formula (III), R₇ and R₈ are preferably an alkyl group or an aryl group.

From the viewpoint of the ease of the synthesis of the raw material monomer, in the above formula (III), R₃ and R4 are preferably an alkyl group, an alkoxy group, an alkylthio group, an aryl group, an aryloxy group, an arylthio group, or a heterocyclic thio group, more preferably an alkyl group, an alkoxy group, an aryl group, or an aryloxy group, and further preferably an aryl group, an alkoxy group, or an aryl group.

From the viewpoint of the ease of the synthesis of the raw material monomer, in the above formula (III), R₁, R₂, R₅, and R₆ are preferably an alkyl group or an alkoxy group.

Specific examples of the repeating unit represented by the above formula (III) include repeating units represented by the following formulas (III-1) to (III-6):

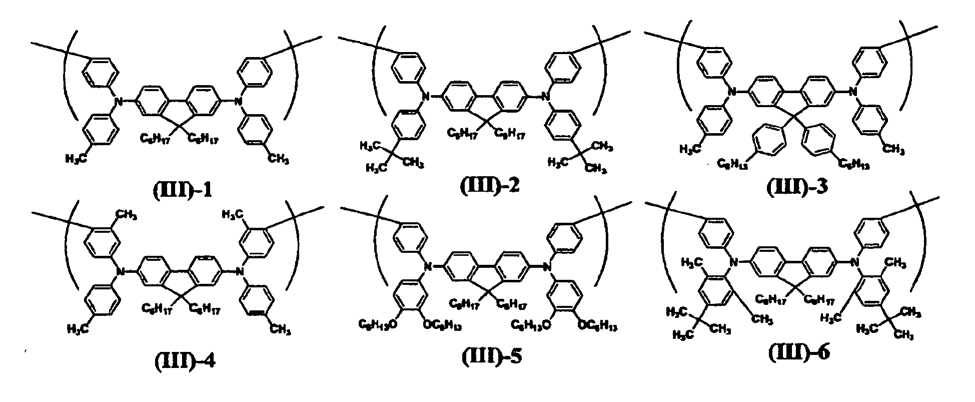

### <Repeating Unit Represented by Formula (IV)>

The block copolymer of the present invention has a repeating unit represented by the following formula (IV), in addition to the repeating units represented by the above formula (II) and the above formula (III), in the block (B). The block copolymer of the present invention may have two or more different types of repeating units represented by the following formula (IV) in the block (B).

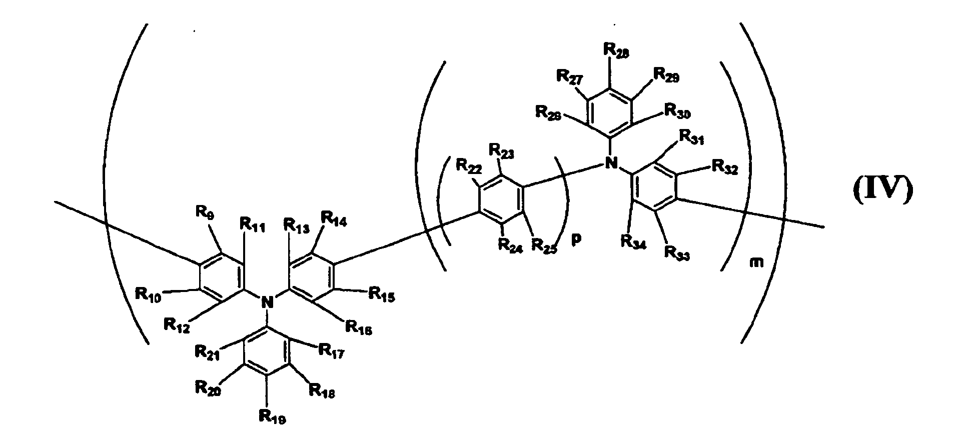

wherein R₉ to R₃₄ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and
m and p independently represent 0 or 1,
provided that at least one of combinations of R₁₁ and R₁₃, R₁₂ and R₂₁, R₂₅ and R₃₄, and R₃₀ and R₃₁ may be taken together to form a single bond or a divalent group represented by the formula: -O- or the formula: - S-, instead of representing the above atom or group.

From the viewpoint of the ease of the synthesis of the raw material monomer, R₉ to R₃₄ are preferably independently a hydrogen atom, an alkyl group, an alkoxy group, an alkylthio group, an aryl group, an aryloxy group, an arylthio group, or a heterocyclic thio group, more preferably a hydrogen atom, an alkyl group, an alkoxy group, an aryl group, or an aryloxy group, and further preferably a hydrogen atom, an alkyl group, or an alkoxy group.

From the viewpoint of the ease of the synthesis of the raw material monomer, when at least one of combinations of R₁₁ and R₁₃, and R₂₅ and R₃₄ is taken together to form a single bond or a divalent group represented by the formula: -O- or the formula: - S-, instead of representing the above atom or group, the at least one combination preferably forms a divalent group represented by the formula: -O- or the formula: -S-, and further preferably a group represented by the formula: -O-.

From the viewpoint of the ease of the synthesis of the raw material monomer, when at least one of combinations of R₁₂ and R₂₁, and R₃₀ and R₃₁ is taken together to form a single bond or a divalent group represented by the formula: -O- or the formula: - S-, instead of representing the above atom or group, the at least one combination preferably forms a single bond.

Specific examples of the repeating unit represented by the above formula (IV) include repeating units represented by the following formulas (IV-1) to (IV-17):

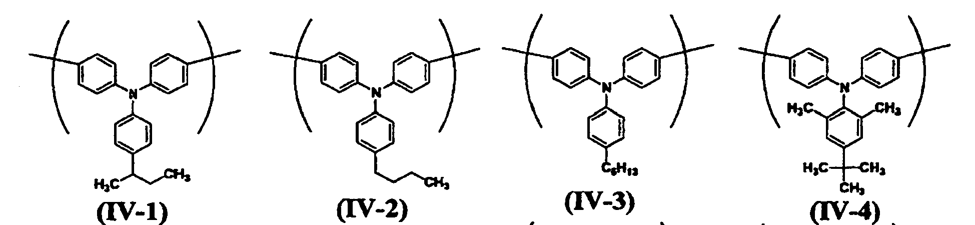

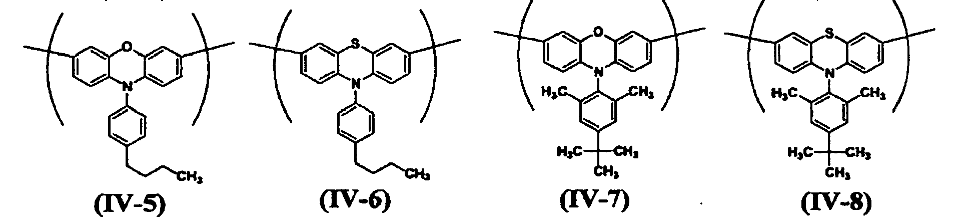

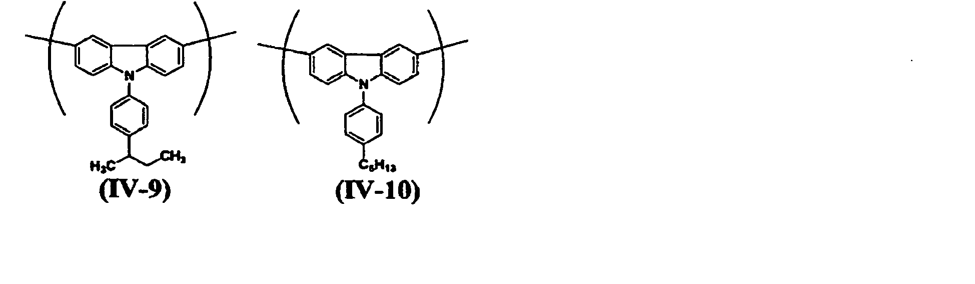

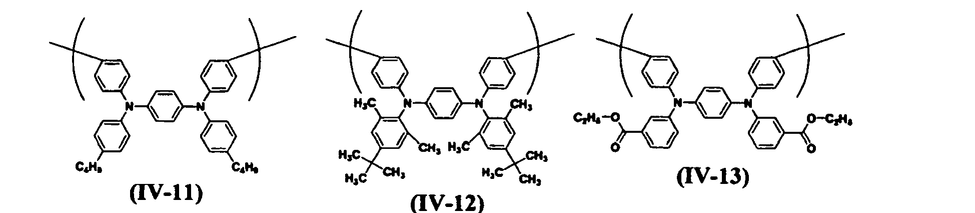

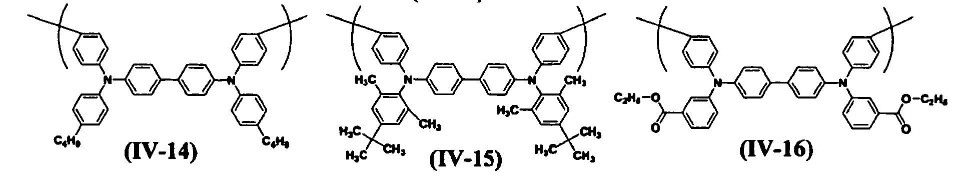

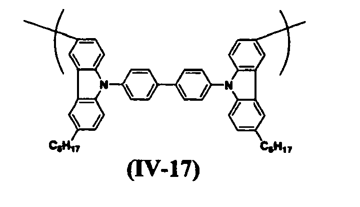

### <Other Repeating Units>

Further, the block copolymer of the present invention may be a copolymer comprising a repeating unit other than the repeating units represented by the above formulas (I) to (IV) in either of the block (A) and the block (B), from the viewpoint of changing the charge transport property, improving heat resistance, and the like. As the repeating unit other than the repeating units represented by the above formulas (I) to (IV), a repeating unit represented by the following formula (C) is preferred.

-Arₐ- (C)

wherein Arₐ represents a divalent heterocyclic group containing no five-membered ring.

The divalent heterocyclic group containing no five-membered ring refers to a remaining atomic group obtained by removing two hydrogen atoms from a heterocyclic compound containing no five-membered ring and means an unsubstituted divalent heterocyclic group containing no five-membered ring, and a substituted divalent heterocyclic group containing no five-membered ring. Here, the heterocyclic compound containing no five-membered ring refers to, among organic compounds having a cyclic structure containing no five-membered ring, one containing, as elements constituting a ring, not only a carbon atom, but also a heteroatom, such as an oxygen atom, a sulfur atom, a nitrogen atom, a phosphorus atom, a boron atom, a silicon atom, a selenium atom, a tellurium atom, and an arsenic atom. Among the divalent heterocyclic groups containing no five-membered ring, a divalent aromatic heterocyclic group containing no five-membered ring is preferred. The substituted atom and substituent in the substituted divalent heterocyclic group containing no five-membered ring are not particularly limited. However, from the viewpoint of solubility, fluorescence characteristics, the ease of synthesis, characteristics when a device is formed, and the like, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group is - preferred. The number of the carbon atoms of a portion excluding the substituent in the divalent heterocyclic group containing no five-membered ring is usually about 3 to 60, preferably about 6 to 40, and more preferably about 6 to 30. Also, the total number of the carbon atoms of the divalent heterocyclic group containing no five-membered ring, including the substituent, is usually about 3 to 100.

The divalent heterocyclic group containing no five-membered ring includes, for example, the following:
divalent six-membered ring-containing heterocyclic groups containing nitrogen as a heteroatom: a pyridine-diyl group (the following formulas 101 to 106), a diazaphenylene group (the following formulas 107 to 110), a quinolinediyl group (the following formulas 111 to 125), a quinoxalinediyl group (the following formulas 126 to 130), an acridinediyl group (the following formulas 131 to 134), a bipyridyldiyl group (the following formulas 135 to 137), and a phenanthrolinediyl group (the following formulas 138 to 140), and
other divalent six-membered ring-containing heterocyclic groups containing oxygen, nitrogen, or the like, as a heteroatom (the following formulas 203 to 206).

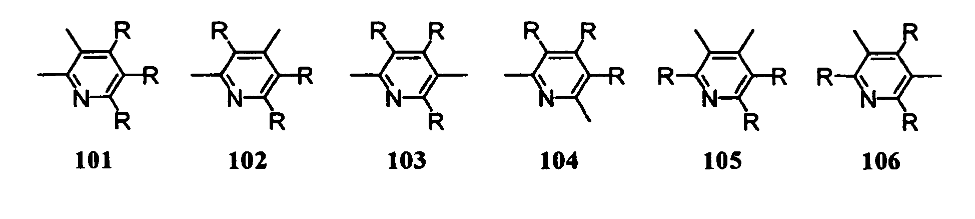

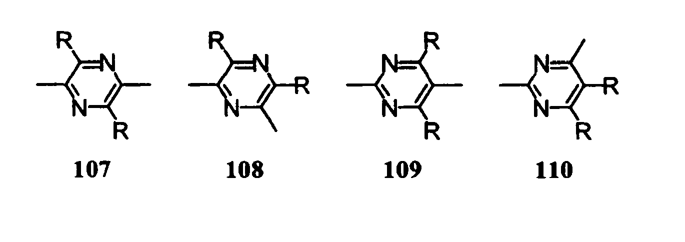

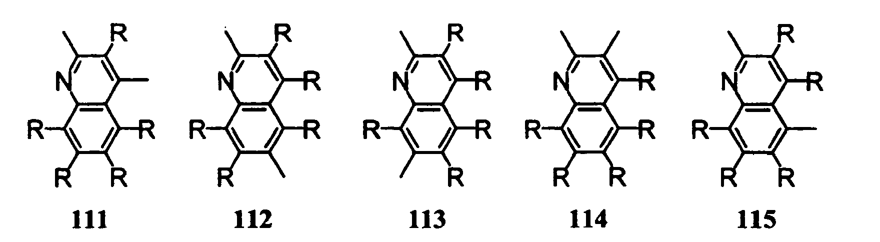

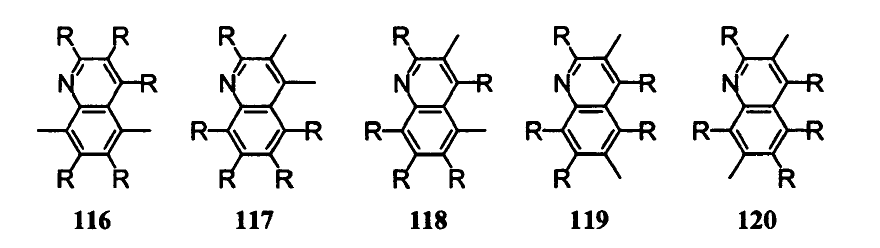

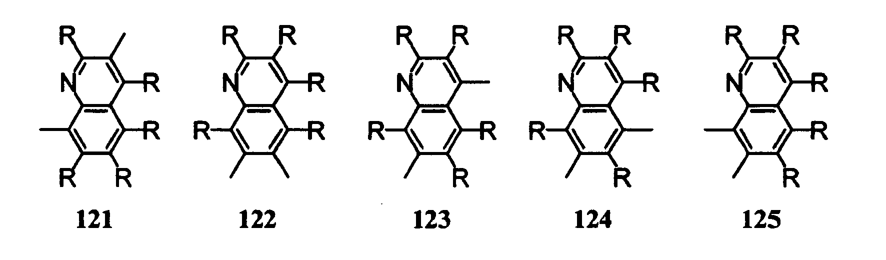

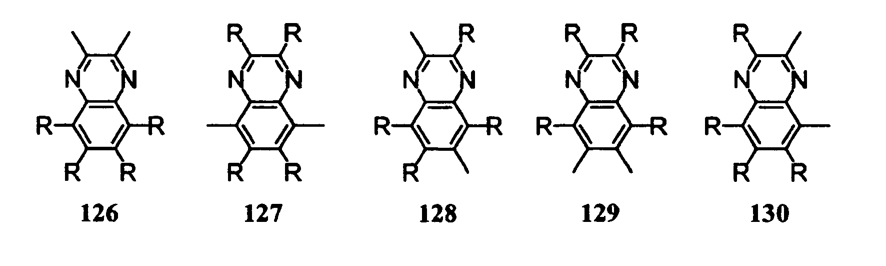

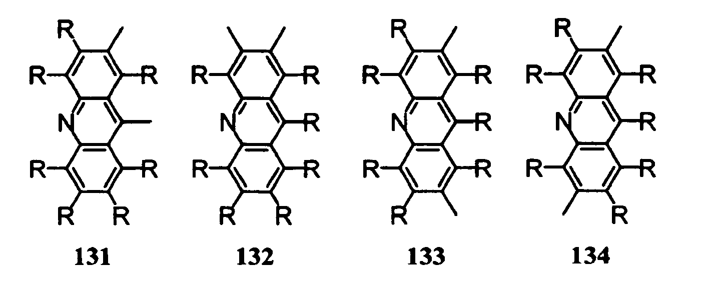

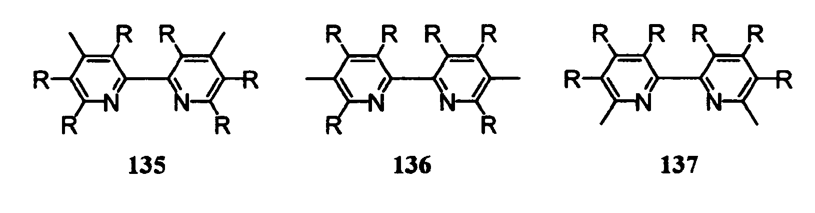

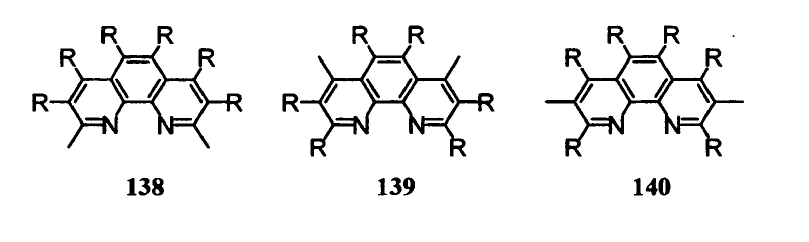

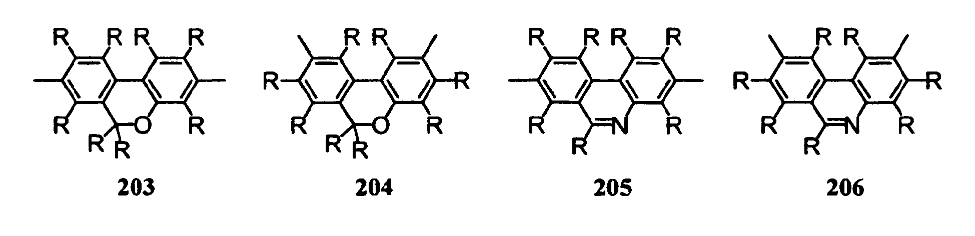

In the above formulas 101 to 140 and 203 to 206, R represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group.

### <Block Copolymer>

With respect to all repeating units in the block copolymer, the content of the total repeating units represented by the above formula (I) is usually in the range of 0.02 mol % to 30 mol %, the content of the repeating unit represented by the above formula (II) is usually in the range of 10 mol % to 90 mol %, the content of the repeating unit represented by the above formula (III) is usually in the range of 0.1 mol % to 30 mol %, more preferably in the range of 0.5 mol % to 20 mol %, and further preferably in the range of 0.5 mol % to 10 mol %, and the content of the repeating unit represented by the above formula (IV) is usually in the range of 0.1 mol % to 50 mol %. Also, when the block copolymer of the present invention comprises a repeating unit other than the repeating units represented by the above formulas (I) to (IV), for example, the repeating unit represented by the above formula (C), the content of such a repeating unit is usually in the range of 0.1 mol % to 30 mol % with respect to all repeating units in the block copolymer.

The block copolymer of the present invention preferably has a polystyrene equivalent number average molecular weight of 1 × 10³ to 1 × 10⁷, and further preferably 1 × 10⁴ to 1 × 10⁷, from the viewpoint of the life characteristics of the device.

The block copolymer of the present invention preferably has a polystyrene equivalent weight average molecular weight of 1 × 10³ to 1 × 10⁷, and further preferably 1 × 10⁴ to 1 × 10⁷, from the viewpoint of the life characteristics of the device.

In the block copolymer of the present invention, the block (A) preferably has a polystyrene equivalent number average molecular weight of 1 × 10³ to 1 × 10⁵, and more preferably 1 × 10⁴ to 1 × 10⁵, from the viewpoint of the life characteristics of the device.

In the block copolymer of the present invention, the block (A) preferably has a polystyrene equivalent weight average molecular weight of 1 × 10³ to 1 × 10⁵, and more preferably 1 × 10° to 1 × 10⁵, from the viewpoint of the life characteristics of the device.

In the present invention, the number average molecular weight and weight average molecular weight based on polystyrene standards are obtained by a size exclusion chromatograph (SEC)(LC-10Avp manufactured by SHIMADZU CORPORATION). The block copolymer to be measured is dissolved in tetrahydrofuran at a concentration of about 0.5 wt%, and 50 µL of the solution is injected into the SEC. Tetrahydrofuran is used as the mobile phase of the SEC and flowed at a flow rate of 0.6 mL/min. As the column, two of TSKgel SuperHM-H (manufactured by TOSOH CORPORATION) and one of TSKgel SuperH2000 (manufactured by TOSOH CORPORATION) are used, connected in series. For the detector, a differential refractive index detector (RID-10A manufactured by SHIMADZU CORPORATION) is used.

When with respect to the total of the blocks (A) and (B), the mole fraction of the block (A) is represented as [A], and the mole fraction of the block (B) is represented as [B], [A]/[B] is preferably 0.1 or more and 10 or less, and more preferably 0.3 or more and 3 or less, from the viewpoint of balancing the electron transport property and hole transport property of the polymer compound of the present invention to increase luminous efficiency.

Also, if a group involved in condensation polymerization remains as it is at the end of the block copolymer of the present invention, the emission characteristics and life when a device is formed may decrease, therefore, the end of the block copolymer may be protected by a stable protective group. As the protective group, one having a conjugated bond continuous with the conjugated structure of the main chain is preferred, and the protective group includes, for example, one bonded to an aryl group or a heterocyclic group via a carbon-carbon bond. Specifically, substituents described in Formula 10 in JP-A-9-45478, and the like are illustrated.

As the good solvent for the block copolymer of the present invention, chloroform, methylene chloride, dichloroethane, tetrahydrofuran, toluene, xylene, mesitylene, tetralin, decalin, n-butylbenzene, and the like are illustrated. Although depending on the structure and molecular weight of the block copolymer, usually, 0.1% or more by weight of the block copolymer can be dissolved in these solvents.

### <Method for Manufacturing Block Copolymer>

Next, a method for manufacturing the block copolymer of the present invention will be described.
The method for synthesizing the block polymer includes, for example, a method in which the block (A) having a high molecular weight is synthesized and a monomer constituting the block (B) is added to the block (A) for polymerization, and a method in which the block (A) having a high molecular weight and the block (B) having a high molecular weight are previously synthesized and these are polymerized together.

For example, the block copolymer of the present invention can be manufactured by using a compound represented by Y₁-Ar₁-Y₂, a compound represented by Y₁-Ar₂-Y₂, a compound represented by Y₁-AY₂, and a compound represented by Y₁-B-Y₂, as raw materials, and subjecting these to condensation polymerization, wherein -A- represents the repeating unit represented by the above formula (III), -B-represents the repeating unit represented by the above formula (IV), Ar₁ and Ar₂ represent the same meaning as the above, and Y₁ and Y₂ each independently represent a substituent involved in condensation polymerization.

Also, when the block copolymer of the present invention has a repeating unit other than the repeating units represented by the above formulas (I) to (IV), a compound having two substituents involved in condensation polymerization and providing the repeating unit other than the repeating units represented by the above formulas (I) to (IV) after polymerization should be coexisted for condensation polymerization. As such a compound, a compound of Y₃-Arₐ-Y₄ is illustrated, wherein Arₐ represents the same meaning as the above, Y₃ and Y₄ represent a substituent involved in condensation polymerization. Specific examples of the compound include a compound represented by the following formula (H). This compound has two substituents involved in condensation polymerization and provides a repeating unit comprising the group represented by the above formula 140 after polymerization.

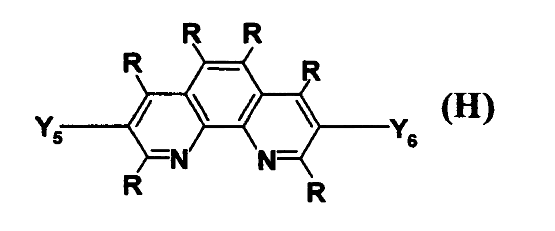

wherein R represents the same meaning as the above, Y₅ and Y₆ represent a substituent involved in condensation polymerization.

In the manufacturing method of the present invention, the substituents (Y₁, Y₂, Y₃, Y₄, Y₅, and Y₆) involved in condensation polymerization include a halogen atom, an alkylsulfo group, an arylsulfo group, an arylalkylsulfo group, a borate group, a sulfonium methyl group, a phosphonate methyl group, a phosphonate methyl group, a methyl monohalide group, a -B(OH)₂, a formyl group, a cyano group, or a vinyl group.

As the alkylsulfo group, a methanesulfo group, an ethanesulfo group, a trifluoromethanesulfo group, and the like are illustrated. As the arylsulfo group, a benzenesulfo group, a p-toluenesulfo group, and the like are illustrated. As the arylalkylsulfo group, a benzylsulfo group and the like are illustrated.

As the borate group, groups represented by the following formulas are illustrated:

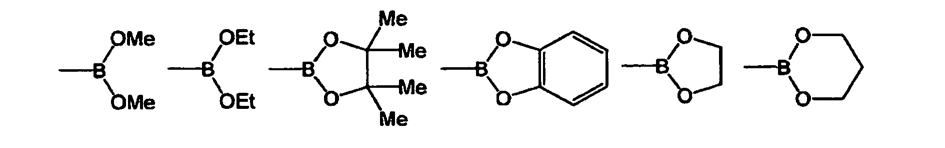

wherein Me represents a methyl group, and Et represents an ethyl group.

As the sulfonium methyl group, groups represented by the following formulas are illustrated:

-CH₂S⁺Me₂X⁻, -CH₂S⁺Ph₂X⁻

wherein X represents a halogen atom, Me represents a methyl group, and Ph represents a phenyl group.

As the phosphonate methyl group, a group represented by the following formula is illustrated:

-CH₂P⁺Ph₃X⁻

wherein X represents a halogen atom, and Ph represents a phenyl group.

As the phosphonate methyl group, a group represented by the following formula is illustrated:

-CH₂PO(OR')₂

wherein R' represents an alkyl group, an aryl group, or an arylalkyl group.

As the methyl monohalide group, a methyl fluoride group, a methyl chloride group, a methyl bromide group, or a methyl iodide group is illustrated.

The substituents preferred as the substituents involved in condensation polymerization are different depending on the type of polymerization reaction, and include a halogen atom, an alkylsulfo group, an arylsulfo group, or an arylalkylsulfo group when, for example, a zero-valent nickel complex (Ni(0) complex) is used, such as in Yamamoto coupling reaction. Also, the substituents include an alkylsulfo group, a halogen atom, a borate group, and -B(OH)₂ when a nickel catalyst or a palladium catalyst is used, such as in Suzuki coupling reaction.

Specifically, the manufacturing method of the present invention can be performed by dissolving a compound having two substituents involved in condensation polymerization, which provides a monomer, in an organic solvent, as required, using, for example, an alkali or a suitable catalyst, and at a temperature equal to or higher than the melting point of the organic solvent and equal to or lower than its boiling point. For example, publicly known methods described in "Organic Reactions", Vol. 14, p. 270-490, John Wiley & Sons, Inc., 1965, "Organic Syntheses", Collective Volume VI, p. 407-411, John Wiley & Sons, Inc., 1988, Chem. Rev., Vol. 95, p. 2457 (1995), J. Organomet. Chem., Vol. 576, p. 147 (1999), Macromol. Chem., Macromol. Symp., Vol. 12, p. 229 (1987), and the like can be used.

As a method for manufacturing a block polymer, for example, publicly known methods described in WO2005/36666 and the like can be used.

In the method for manufacturing the block copolymer of the present invention, known condensation reaction can be used according to the substituents involved in condensation polymerization. For example, a method for polymerizing the monomer concerned by Suzuki coupling reaction, a method for polymerizing the monomer concerned by Grignard reaction, a method for polymerizing the monomer concerned by a zero-valent nickel complex, a method for polymerizing the monomer concerned by an oxidant, such as FeCl₃, a method for electrochemically oxidatively polymerizing the monomer concerned, or a method using the decomposition of an intermediate polymer having a suitable leaving group, and the like are illustrated. Among these, a method for polymerizing by Suzuki coupling reaction, a method for polymerizing by Grignard reaction, and a method for polymerizing by a zero-valent nickel complex are preferred because of easy structure control.

Among the manufacturing methods of the present invention, a manufacturing method is preferred, in which the substituents (Y₁, Y₂, Y₃, Y₄, Y₅, and Y₆) involved in condensation polymerization are independently selected from a halogen atom, an alkylsulfo group, an arylsulfo group, or an arylalkylsulfo group, and condensation polymerization is performed in the presence of a zero-valent nickel complex. The raw material compound includes, for example, a dihalogenated compound, a bis(alkylsulfonate) compound, a bis(arylsulfonate) compound, a bis(arylalkylsulfonate) compound, a halogen-alkylsulfonate compound, a halogen-arylsulfonate compound, a halogen-arylalkylsulfonate compound, an alkylsulfonate-arylsulfonate compound, an alkylsulfonate-arylalkylsulfonate compound, and an arylsulfonate-arylalkylsulfonate compound. A method for manufacturing a sequence-controlled block copolymer by using, among these, for example, a halogen-alkylsulfonate compound, a halogen-arylsulfonate compound, a halogen-arylalkylsulfonate compound, an alkylsulfonate-arylsulfonate compound, an alkylsulfonate-arylalkylsulfonate compound, or an arylsulfonate-arylalkylsulfonate compound, as the raw material compound, is mentioned.

Also, among the manufacturing methods of the present invention, a manufacturing method is preferred, in which the substituents (Y₁, Y₂, Y₃, Y₄, Y₅, and Y₆) involved in condensation polymerization are independently selected from a halogen atom, an alkylsulfo group, an arylsulfo group, an arylalkylsulfo group, a boric acid group (-B(OH)₂), or a borate group, the ratio of the total mole number of the halogen atom, alkylsulfo group, arylsulfo group, and arylalkylsulfo group of all raw material compounds (J) and the total mole number of the boric acid group and borate group of all raw material compounds (K), K/J, is substantially 1 (usually in the range of 0.7 to 1.2), and condensation polymerization is performed using a nickel catalyst or a palladium catalyst. Specific combinations of raw material compounds include a combination of a dihalogenated compound, a bis(alkylsulfonate) compound, a bis(arylsulfonate) compound, or a bis(arylalkylsulfonate) compound, and a diboric acid compound or a diborate compound, and a halogen-boric acid compound, a halogen-borate compound, an alkylsulfonate-boric acid compound, an alkylsulfonate-borate compound, an arylsulfonate-boric acid compound, an arylsulfonate-borate compound, an arylalkylsulfonate-boric acid compound, and an arylalkylsulfonate-borate compound. A method for manufacturing a sequence-controlled block copolymer by using, among these, for example, a halogen-boric acid compound, a halogen-borate compound, an alkylsulfonate-boric acid compound, an alkylsulfonate-borate compound, an arylsulfonate-boric acid compound, an arylsulfonate-borate compound, an arylalkylsulfonate-boric acid compound, or an arylalkylsulfonate-borate compound, as the raw material compound, is mentioned.

The solvent used in reaction is different depending on the compound used and reaction and is generally preferably subjected to sufficient deoxidation treatment to suppress side reaction. Preferably, reaction is allowed to proceed in an inert atmosphere. Also, similarly, the solvent used in reaction is preferably subjected to dehydration treatment.
However, this does not apply to reaction in a two-phase system with water, such as Suzuki coupling reaction.

As the solvent, saturated hydrocarbons, such as pentane, hexane, heptane, octane, cyclohexane, and decalin, aromatic hydrocarbons, such as benzene, toluene, ethylbenzene, n-butylbenzene, xylene, and tetralin, halogenated saturated hydrocarbons, such as carbon tetrachloride, chloroform, dichloromethane, chlorobutane, bromobutane, chloropentane, bromopentane, chlorohexane, bromohexane, chlorocyclohexane, and bromocyclohexane, halogenated aromatic hydrocarbons, such as chlorobenzene, dichlorobenzene, and trichlorobenzene, alcohols, such as methanol, ethanol, propanol, isopropanol, butanol, and t-butyl alcohol, carboxylic acids, such as formic acid, acetic acid, and propionic acid, ethers, such as dimethyl ether, diethyl ether, methyl-t-butyl ether, tetrahydrofuran, tetrahydropyran, and dioxane, amines, such as trimethylamine, triethylamine, N,N,N',N'-tetramethylethylenediamine, and pyridine, amides, such as N,N-dimethylformamide, N,N-dimethylacetamide, N,N-diethylacetamide, and N-methylmorpholine oxide, and the like are illustrated. These solvents may be used alone, or mixed and used. Among these, ethers are preferred, and tetrahydrofuran and diethyl ether are further preferred.

For reaction, an alkali or a suitable catalyst is appropriately added. These should be selected according to reaction used. The alkali or the catalyst is preferably one that is sufficiently dissolved in the solvent used in reaction. The alkali includes, for example, inorganic bases, such as potassium carbonate and sodium carbonate; organic bases, such as triethylamine; and inorganic salts, such as cesium fluoride. The catalyst includes, for example, palladium[tetrakis(triphenylphosphine)] and palladium acetates. As a method for mixing the alkali or the catalyst, a method for slowly adding a solution of the alkali or the catalyst while stirring a reaction solution in an inert atmosphere, such as argon and nitrogen, or on the contrary a method for slowly adding a reaction solution to a solution of the alkali or the catalyst is illustrated.

When the block copolymer of the present invention is used in a polymer LED or the like, preferably, polymerization is performed after a monomer before polymerization is purified by a method, such as distillation, sublimation purification, and recrystallization, because the purity of the block copolymer affects the performance of the device, such as emission characteristics. Also, after polymerization, preferably, purification treatment, such as reprecipitation purification, and fractionation by chromatography, is performed.

### <Composition>

The composition of the present invention is a composition containing:
the block copolymer of the present invention; and
a solvent, a light-emitting material other than the block copolymer, a hole transport material other than the block copolymer, or an electron transport material other than the block copolymer, or a combination of two or more thereof. The composition may contain at least two types of the block copolymers.

The solvent can be added to turn the composition of the present invention into a liquid composition described later. As the solvent included in the composition, one in which components other than the solvent in the composition can be dissolved or dispersed is preferred. As the solvent, chlorine solvents, such as chloroform, methylene chloride, 1,2-dichloroethane, 1,1,2-trichloroethane, chlorobenzene, and o-dichlorobenzene, ether solvents, such as tetrahydrofuran and dioxane, aromatic hydrocarbon solvents, such as toluene, xylene, trimethylbenzene, and mesitylene, aliphatic hydrocarbon solvents, such as cyclohexane, methylcyclohexane, n-pentane, n-hexane, n-heptane, n-octane, n-nonane, and n-decane, ketone solvents, such as acetone, methyl ethyl ketone, and cyclohexanone, ester solvents, such as ethyl acetate, butyl acetate, methyl benzoate, and ethyl cellosolve acetate, polyalcohols, such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof, alcohol solvents, such as methanol, ethanol, propanol, isopropanol, and cyclohexanol, sulfoxide solvents, such as dimethylsulfoxide, and amide solvents, such as N-methyl-2-pyrrolidone and N,N-dimethylformamide, are illustrated. Also, one of these solvents may be used alone, or a plurality of these solvents may be combined and used. It is preferred to comprise, among the solvents, one or more types of organic solvents having a structure containing at least one or more benzene rings and having a melting point of 0°C or less and a boiling point of 100°C or more, from the viewpoint of viscosity, a film formation property, and the like.

For the type of the solvent, from the viewpoint of the solubility of the components other than the solvent in the composition in the solvent, uniformity during film formation, viscosity characteristics, and the like, aromatic hydrocarbon solvents, aliphatic hydrocarbon solvents, ester solvents, and ketone solvents are preferred, and specifically, toluene, xylene, ethylbenzene, diethylbenzene, trimethylbenzene, mesitylene, n-propylbenzene, isopropylbenzene, n-butylbenzene, isobutylbenzene, s-butylbenzene, anisole, ethoxybenzene, 1-methylnaphthalene, cyclohexane, cyclohexanone, cyclohexylbenzene, bicyclohexyl, cyclohexenylcyclohexanone, n-heptylcyclohexane, n-hexylcyclohexane, methyl benzoate, 2-propylcyclohexanone, 2-heptanone, 3-heptanone, 4-heptanone, 2-octanone, 2-nonanone, 2-decanone, and dicyclohexyl ketone are preferred. It is more preferred to comprise at least one type of xylene, anisole, mesitylene, cyclohexylbenzene, and bicyclohexylmethyl benzoate.

For the type of the solvent included in the composition, two or more types are preferred, two to three types are more preferred, and two types are further preferred, from the viewpoint of a film formation property, device characteristics, and the like.

When two types of solvents are included in the composition, one type of solvent of these may be in a solid state at 25°C. From the viewpoint of a film formation property, preferably, one type of solvent has a boiling point of 180°C or more, and the other type of solvent has a boiling point of less than 180°C, and more preferably, one type of solvent has a boiling point of 200°C or more, and the other type of solvent has a boiling point of less than 180°C. Also, from the viewpoint of viscosity, preferably, 0.2% or more by weight of the components in the composition excluding the solvents are dissolved in the solvents at 60°C, and preferably, 0.2% or more by weight of the components in the composition excluding the solvents are dissolved in one type of solvent of the two types of solvents at 25°C.

When three types of solvents are included in the composition, one or two types of solvents of these may be in a solid state at 25°C. From the viewpoint of a film formation property, preferably, at least one type of solvent of the three types of solvents is a solvent having a boiling point of 180°C or more, and at least one type of solvent is a solvent having a boiling point of less than 180°C, and more preferably, at least one type of solvent of the three types of solvents is a solvent having a boiling point of 200°C or more and 300°C or less, and at least one type of solvent is a solvent having a boiling point of less than 180°C. Also, from the viewpoint of viscosity, preferably, 0.2% or more by weight of the components in the composition excluding the solvents are dissolved in two types of solvents of the three types of solvents at 60°C, and preferably, 0.2% or more by weight of the components in the composition excluding the solvents are dissolved in one type of solvent of the three types of solvents at 25°C.

When two or more types of solvents are included in the composition, the solvent having the highest boiling point is preferably 40 to 90 wt% of the weight of all solvents included in the composition, more preferably 50 to 90% by weight, and further preferably 65 to 85% by weight, from the viewpoint of viscosity and a film formation property.

The block copolymer of the present invention can be used as the light-emitting material, hole transport material, and electron transport material of a light-emitting device, as described above. However, at least one of other light-emitting materials, hole transport materials, and electron transport materials can be added, as required, to modify the characteristics of the obtained light-emitting device.

The light-emitting material other than the block copolymer includes, for example, a low-molecular fluorescent material. Its specific examples include fluorescent materials of low-molecular compounds, such as naphthalene derivatives, anthracene, anthracene derivatives, perylene, perylene derivatives, dyes, such as a polymethine dye, a xanthene dye, a coumarin dye, and a cyanine dye, a metal complex having 8-hydroxyquinoline as a ligand, a metal complex having an 8-hydroxyquinoline derivative as a ligand, other fluorescent metal complexes, aromatic amine, tetraphenylcyclopentadiene, tetraphenylcyclopentadiene derivatives, tetraphenylbutadiene, tetraphenylbutadiene derivatives, stilbene, silicon-containing aromatic compounds, oxazole, furoxan, thiazole, tetraarylmethane, thiadiazole, pyrazole, metacyclophane, and acetylene, and specifically, publicly known ones, for example, those described in JP-A-57-51781 and JP-A-59-194393. One of these light-emitting materials may be used alone, or two or more of these light-emitting materials may be used in combination.

The hole transport material other than the block copolymer includes, for example, polyvinylcarbazole and its derivatives, polysilane and its derivatives, polysiloxane derivatives having aromatic amine in the side chain or the main chain, pyrazoline derivatives, arylamine derivatives, stilbene derivatives, triphenyldiamine derivatives, polyaniline and its derivatives, polythiophene and its derivatives, polypyrrole and its derivatives, poly(p-phenylenevinylene) and its derivatives, and poly(2,5-thienylenevinylene) and its derivatives. One of these hole transport materials may be used alone, or two or more of these hole transport materials may be used in combination.

The electron transport material other than the block copolymer includes, for example, oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, and polyfluorene and its derivatives. One of these electron transport materials may be used alone, or two or more of these electron transport materials may be used in combination.

The composition of the present invention may comprise a stabilizer, an additive for adjusting viscosity and/or surface tension, an antioxidant, and the like, as other optional components, as required, other than the block copolymer, the solvent, the light-emitting material other than the block copolymer, the hole transport material other than the block copolymer, and the electron transport material other than the block copolymer. One of each of these optional components may be used alone, or two or more of each of these optional components may be used in combination.

The stabilizer that may be contained in the composition of the present invention includes, for example, a phenol antioxidant and a phosphorus antioxidant.

The additive for adjusting viscosity and/or surface tension that may be contained in the composition of the present invention includes, for example, a high-molecular-weight compound (thickener) and a poor solvent for increasing viscosity, a low-molecular-weight compound for decreasing viscosity, and a surfactant for decreasing surface tension, and a combination thereof.

The high-molecular-weight compound should be one not inhibiting emission or charge transport, and when the composition comprises a solvent, the high-molecular-weight compound is usually one soluble in the solvent. As the high-molecular-weight compound, for example, high-molecular-weight polystyrene, high-molecular-weight polymethyl methacrylate, and the like can be used. The high-molecular-weight compound preferably has a polystyrene equivalent weight average molecular weight of 500,000 or more, and more preferably 1,000,000 or more. Also, a poor solvent can be used as a thickener.

The antioxidant that may be contained in the composition of the present invention should be one not inhibiting emission or charge transport, and when the composition comprises a solvent, the antioxidant is usually one soluble in the solvent.
As the antioxidant, a phenol antioxidant, a phosphorus antioxidant, and the like are illustrated. The use of the antioxidant can improve the storage stability of the block copolymer and the solvent.

In this specification, a "liquid composition" means one that comprises the block copolymer and a solvent and is liquid during device fabrication, typically, at normal pressure (that is, 1 atmospheric pressure) and 25°C. The liquid composition may be generally called an ink, an ink composition, a solution, or the like. The liquid composition of the present invention is useful for the fabrication of a light-emitting device, such as a polymer light-emitting device, and an organic transistor.

When a film is formed using this liquid composition in fabricating a polymer light-emitting device, after coating with the liquid composition, the solvent need only be removed by drying. Also, when the light-emitting material, the hole transport material, or the charge transport material is mixed, a similar method can be applied. Therefore, such a film formation is very advantageous in manufacture. In drying, drying may be performed with the temperature increased to about 50 to 150°C, and drying may be performed with the pressure reduced to about 10⁻³ Pa.

As the film formation method using the liquid composition, coating methods, such as a spin coating method, a casting method, a microgravure coating method, a gravure coating method, a bar coating method, a roll coating method, a wire bar coating method, a dip coating method, a slit coating method, a capillary coating method, a spray coating method, a screen printing method, a flexographic printing method, an offset printing method, an ink jet printing method, and a nozzle coating method, can be used. Printing methods, such as a screen printing method, a flexographic printing method, an offset printing method, and an ink jet printing method, are preferred in that pattern formation and the separate application of many colors are easy.

The viscosity of the liquid composition is different depending on the printing method and is preferably in the range of 0.5 to 500 mPa·s at 25°C. In the case of a printing method in which the liquid composition passes through an ejection apparatus, such as an ink jet printing method, the viscosity is preferably in the range of 0.5 to 20 mPa·s at 25°C to prevent clogging and flight bending during ejection.

The ratio of the block copolymer of the present invention in the composition of the present invention is usually 1% to 99.9% by weight, preferably 10% to 99.9% by weight, and further preferably 20% to 99.9% by weight, with respect to the total weight of the composition.

When the composition of the present invention contains a solvent, the ratio of the solvent in the composition is usually 1% to 99.9% by weight, preferably 60% to 99.9% by weight, and further preferably 90% to 99.8% by weight, with respect to the total weight of the composition.

When the composition of the present invention contains a light-emitting material other than the block copolymer of the present invention, the ratio of the light-emitting material in the composition is usually 1% to 80% by weight, and preferably 5% to 60% by weight, with respect to the total weight of the composition.

When the composition of the present invention contains a hole transport material other than the block copolymer of the present invention, the ratio of the hole transport material in the composition is usually 1% to 80% by weight, and preferably 5% to 60% by weight, with respect to the total weight of the composition.

When the composition of the present invention contains an electron transport material other than the block copolymer of the present invention, the ratio of the electron transport material in the composition is usually 1% to 80% by weight, and preferably 5% to 60% by weight, with respect to the total weight of the composition.

In the composition of the present invention, the total of the ratio of the block copolymer of the present invention, the ratio of the solvent, the light-emitting material other than the block copolymer, the hole transport material other than the block copolymer, or the electron transport material other than the block copolymer, or a combination of two or more thereof, and the ratio of the above other optional components added as required is 100 wt% with respect to the total weight of the composition.

### <Use>

The block copolymer of the present invention can be used not only as a light-emitting material, but also as a thin film, an organic semiconductor material, an organic transistor, an optical material, or a solar cell, or as a conductive material by doping.

The thin film of the present invention will be described. This thin film is formed by using the block copolymer. As the types of the thin film, a light-emitting thin film, a conductive thin film, an organic semiconductor thin film, and the like are illustrated.

The light-emitting thin film of the present invention contains the block copolymer of the present invention. The light-emitting thin film may further contain a light-emitting material other than the block copolymer, a hole transport material other than the block copolymer, or an electron transport material other than the block copolymer, or a combination of two or more thereof. Examples of these light-emitting material, hole transport material, and electron transport material are as described above. From the viewpoint of the luminance of the device, emission voltage, and the like, the light-emitting thin film preferably has an emission quantum yield of 50% or more, more preferably 60% or more, and further preferably 70% or more.

The conductive thin film preferably has a surface resistance of 1 KΩ/□ or less. Doping the thin film with Lewis acid, an ionic compound, or the like can increase conductivity. The surface resistance is more preferably 100 Ω/□ or less, and further preferably 10 Ω/□ or less.

For the organic semiconductor thin film, either higher one of the electron mobility or the hole mobility is preferably 10⁻⁵ cm²/V second or more, more preferably 10⁻³ cm²/V second or more, and further preferably 10⁻¹ cm²/V second or more. Also, the organic semiconductor thin film can be used to fabricate an organic transistor. Specifically, an organic transistor can be provided by forming the organic semiconductor thin film on a Si substrate on which an insulating film of SiO₂ or the like and a gate electrode are formed and forming a source electrode and a drain electrode with Au or the like.

Next, a polymer field effect transistor, one aspect of the organic transistor, will be described.

The block copolymer of the present invention can be suitably used as a material of a polymer field effect transistor, especially as an active layer. For the structure of the polymer field effect transistor, usually, a source electrode and a drain electrode should be provided in contact with an active layer of a polymer, and a gate electrode should be further provided sandwiching an insulating layer in contact with the active layer.

The polymer field effect transistor is usually formed on a supporting substrate. The material of the supporting substrate is not particularly limited as long as the characteristics of the field effect transistor are not inhibited. As the supporting substrate, a glass substrate, a flexible film substrate, and a plastic substrate can also be used, in addition to the Si substrate.

The polymer field effect transistor can be manufactured by a publicly known method, for example, a method described in JP-A-5-110069.

In forming the active layer, using a solution obtained by dissolving the block copolymer of the present invention in a solvent is very advantageous and preferred in manufacture. As the method for film formation from the solution, coating methods, such as a spin coating method, a casting method, a microgravure coating method, a gravure coating method, a bar coating method, a roll coating method, a wire bar coating method, a dip coating method, a slit coating method, a capillary coating method, a spray coating method, a screen printing method, a flexographic printing method, an offset printing method, an ink jet printing method, and a nozzle coating method, can be used. Printing methods, such as a screen printing method, a flexographic printing method, an offset printing method, and an ink jet printing method, are preferred in that pattern formation and the separate application of many colors are easy.

A sealed polymer field effect transistor obtained by sealing a polymer field effect transistor after fabricating it is preferred. Thus, the polymer field effect transistor is cut off from the air, and a decrease in the characteristics of the polymer field effect transistor can be suppressed.

The sealing method includes a method for covering with an ultraviolet (UV) curing resin, a thermosetting resin, an inorganic SiONx film, or the like, and a method for bonding a glass plate or a film with a UV curing resin, a thermosetting resin, or the like. Preferably, steps after the fabrication of the polymer field effect transistor until sealing are performed without exposure to the air (for example, in a dry nitrogen atmosphere, in a vacuum, and the like) to effectively cut off from the air.

Next, an organic solar cell will be described. A solid photoelectric conversion device that is an organic photoelectric conversion device, one aspect of the organic solar cell, and uses photovoltaic effect will be described.

The block copolymer of the present invention can be suitably used as a material of an organic photoelectric conversion device, especially as the organic semiconductor layer of a Schottky barrier type device using the interface between an organic semiconductor and metal, and also as the organic semiconductor layer of a pn heterojunction type device using the interface between an organic semiconductor and an inorganic semiconductor or organic semiconductors.

Further, the block copolymer of the present invention can be suitably used as an electron-donating polymer and an electron-accepting polymer in a bulk heterojunction type device in which the contact area of the donor and the acceptor is increased, and also as the electron-donating conjugated polymer (dispersed support) of an organic photoelectric conversion device using a polymer and low-molecular compound composite system, for example, a bulk heterojunction type organic photoelectric conversion device in which a fullerene derivative is dispersed as an electron acceptor.

For the structure of the organic photoelectric conversion device, for example, in a pn heterojunction type device, a p-type semiconductor layer should be formed on an ohmic electrode, for example, ITO, an n-type semiconductor layer should be further laminated, and an ohmic electrode should be provided on the n-type semiconductor layer.

The organic photoelectric conversion device is usually formed on a supporting substrate. The material of the supporting substrate is not particularly limited as long as the characteristics of the organic photoelectric conversion device are not inhibited. As the supporting substrate, a glass substrate, a flexible film substrate, and a plastic substrate can also be used, in addition to a Si substrate.

The organic photoelectric conversion device can be manufactured by publicly known methods, for example, a method described in Synth. Met., 102, 982 (1999) and a method described in Science, 270, 1789 (1995).

Next, the polymer light-emitting device of the present invention will be described.

The polymer light-emitting device of the present invention comprises:
an anode;
a cathode; and
an organic layer comprising the block copolymer and provided between the anode and the cathode. The organic layer may further comprise a light-emitting material other than the block copolymer, a hole transport material other than the block copolymer, or an electron transport material other than the block copolymer, or a combination of two or more thereof. Examples of these light-emitting material, hole transport material, and electron transport material are as described above. in the polymer light-emitting device of the present invention, the organic layer is preferably a light-emitting layer.

Also, the polymer light-emitting device of the present invention includes (1) a polymer light-emitting device in which an electron transport layer is provided between a cathode and a light-emitting layer, (2) a polymer light-emitting device in which a hole transport layer is provided between an anode and a light-emitting layer, and (3) a polymer light-emitting device in which an electron transport layer is provided between a cathode and a light-emitting layer, and a hole transport layer is provided between an anode and the light-emitting layer.

More specifically, the following structures a) to d) are illustrated.
a) anode/light-emitting layer/cathode
b) anode/hole transport layer/light-emitting layer/cathode
c) anode/light-emitting layer/electron transport layer/cathode
d) anode/hole transport layer/light-emitting layer/electron transport layer/cathode (Here, "/" represents that layers are laminated adjacent to each other. The same applies hereinafter.)

Here, the light-emitting layer is a layer having the function of emitting light, the hole transport layer is a layer having the function of transporting holes, and the electron transport layer is a layer having the function of transporting electrons. The electron transport layer and the hole transport layer are collectively called a charge transport layer. Two or more light-emitting layers, two or more hole transport layers, and two or more electron transport layers may be used.
Also, the hole transport layer adjacent to the light-emitting layer may also be called an interlayer layer.

The method for forming the light-emitting layer is not limited, and a method of film formation from a solution is illustrated.

As the method for film formation from a solution, coating methods, such as a spin coating method, a casting method, a microgravure coating method, a gravure coating method, a bar coating method, a roll coating method, a wire bar coating method, a dip coating method, a slit coating method, a capillary coating method, a spray coating method, a screen printing method, a flexographic printing method, an offset printing method, an ink jet printing method, and a nozzle coating method, can be used. Printing methods, such as a screen printing method, a flexographic printing method, an offset printing method, and an ink jet printing method, are preferred in that pattern formation and the separate application of many colors are easy.

When a film is formed from a solution by using the block copolymer of the present invention in fabricating the polymer light-emitting device, after coating with this solution, the solvent need only be removed by drying. Also, when the light-emitting material, the hole transport material, or the charge transport material is mixed, a similar method can be applied. Therefore, such a film formation is very advantageous in manufacture.

The optimal value of the film thickness of the light-emitting layer is different depending on the material used, and the film thickness should be selected so that the driving voltage and the luminous efficiency are suitable values. The film thickness of the light-emitting layer is, for example, 1 nm to 1 µm, preferably 2 nm to 500 nm, and further preferably 5 nm to 200 nm.

In the polymer light-emitting device of the present invention, a light-emitting material other than the above block copolymer may be mixed and used for the light-emitting layer. Also, in the polymer light-emitting device of the present invention, a light-emitting layer comprising a light-emitting material other than the above block copolymer may be laminated with a light-emitting layer comprising the above block copolymer.

As the light-emitting material other than the block copolymer, a publicly known one can be used. For low-molecular compounds, for example, naphthalene derivatives, anthracene and its derivatives, perylene and its derivatives, dyes, such as polymethine, xanthene, coumarin, and cyanine, metal complexes of 8-hydroxyquinoline and its derivatives, aromatic amine, tetraphenylcyclopentadiene and its derivatives, tetraphenylbutadiene and its derivatives, and the like can be used.
Specifically, publicly known ones, for example, those described in JP-A-57-51781 and JP-A-59-194393, can be used.

When the polymer light-emitting device of the present invention has the hole transport layer, as the hole transport material used, polyvinylcarbazole and its derivatives, polysilane and its derivatives, polysiloxane derivatives having aromatic amine in the side chain or the main chain, pyrazoline derivatives, arylamine derivatives, stilbene derivatives, triphenyldiamine derivatives, polyaniline and its derivatives, polythiophene and its derivatives, polypyrrole and its derivatives, poly(p-phenylenevinylene) and its derivatives, and poly(2,5-thienylenevinylene) and its derivatives are illustrated. Specifically, as the hole transport material, those described in JP-A-63-70257, JP-A-63-175860, JP-A-2-135359, JP-A-2-135361, JP-A-2-209988, JP-A-3-37992, and JP-A-3-152184, and the like are illustrated.

Among these, as the hole transport material used for the hole transport layer, polymer hole transport materials, such as polyvinylcarbazole and its derivatives, polysilane and its derivatives, polysiloxane derivatives having an aromatic amine compound group in the side chain or the main chain, polyaniline and its derivatives, polythiophene and its derivatives, poly(p-phenylenevinylene) and its derivatives, and poly(2,5-thienylenevinylene) and its derivatives, are preferred, and polyvinylcarbazole and its derivatives, polysilane and its derivatives, and polysiloxane derivatives having aromatic amine in the side chain or the main chain are further preferred. A low-molecular hole transport material is preferably dispersed in a polymer binder and used.

Polyvinylcarbazole and its derivatives are obtained, for example, by the cationic polymerization or radical polymerization of a vinyl monomer.

As polysilane and its derivatives, compounds described in Chem. Rev., Vol. 89, p. 1359 (1989), and British Patent GB2300196, and the like are illustrated. Also for the synthesis method, methods described in these can be used, and particularly, the Kipping method is suitably used.

For polysiloxane derivatives, the siloxane skeleton structure hardly has a hole transport property, so that one having the structure of the above low-molecular hole transport material in the side chain or the main chain is suitably used. Particularly, one having aromatic amine having a hole transport property in the side chain or the main chain is illustrated.

The method for forming the hole transport layer is not limited. For the low-molecular hole transport material, a method of film formation from a mixed solution of the low-molecular hole transport material and a polymer binder is illustrated. Also, for the polymer hole transport material, a method of film formation from a solution is illustrated.

The solvent used for film formation from a solution is not particularly limited as long as the hole transport material is dissolved in the solvent.
As the solvent, chlorine solvents, such as chloroform, methylene chloride, and dichloroethane, ether solvents, such as tetrahydrofuran, aromatic hydrocarbon solvents, such as toluene and xylene, ketone solvents, such as acetone and methyl ethyl ketone, and ester solvents, such as ethyl acetate, butyl acetate, and ethyl cellosolve acetate, are illustrated.

As the method for film formation from a solution, coating methods, such as a spin coating method, a casting method, a microgravure coating method, a gravure coating method, a bar coating method, a roll coating method, a wire bar coating method, a dip coating method, a slit coating method, a capillary coating method, a spray coating method, a screen printing method, a flexographic printing method, an offset printing method, an ink jet printing method, and a nozzle coating method, can be used. Printing methods, such as a screen printing method, a flexographic printing method, an offset printing method, and an ink jet printing method, are preferred in that pattern formation and the separate application of many colors are easy.

As the polymer binder mixed, one not extremely inhibiting charge transport is preferred, and also, one not strongly absorbing visible light is suitably used. As the polymer binder, polycarbonate, polyacrylate, polymethyl acrylate, polymethyl methacrylate, polystyrene, polyvinyl chloride, polysiloxane, and the like are illustrated.

The optimal value of the film thickness of the hole transport layer is different depending on the material used, and the film thickness should be selected so that the driving voltage and the luminous efficiency are suitable values. However, at least a thickness such that no pinholes occur is necessary. If the thickness is too thick, the driving voltage of the device is high, so that too thick thickness is not preferred. Therefore, the film thickness of the hole transport layer is, for example, 1 nm to 1 µm, preferably 2 nm to 500 nm, and further preferably 5 nm to 200 nm.

When the polymer light-emitting device of the present invention has the electron transport layer, as the electron transport material used, a publicly known one can be used, and oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, and polyfluorene and its derivatives are illustrated. Specifically, those described in JP-A-63-70257, JP-A-63-175860, JP-A-2-135359, JP-A-2-135361, JP-A-2-209988, JP-A-3-37992, and JP-A-3-152184, and the like are illustrated.

Among these, oxadiazole derivatives, benzoquinone and its derivatives, anthraquinone and its derivatives, metal complexes of 8-hydroxyquinoline and its derivatives, polyquinoline and its derivatives, polyquinoxaline and its derivatives, and polyfluorene and its derivatives are preferred, and 2-(4-biphenylyl)-5-(4-t-butylphenyl)-1,3,4-oxadiazole, benzoquinone, anthraquinone, tris(8-quinolinol)aluminum, and polyquinoline are further preferred.

The method for forming the electron transport layer is not particularly limited. For the low-molecular electron transport material, a method for vacuum deposition from a powder, and a method of film formation from a solution or a melted state are illustrated. For the polymer electron transport material, a method of film formation from a solution or a melted state is illustrated. During film formation from a solution or a melted state, a polymer binder may be used in combination.

The solvent used for film formation from a solution is not particularly limited as long as the electron transport material and/or the polymer binder are dissolved in the solvent. As the solvent, chlorine solvents, such as chloroform, methylene chloride, and dichloroethane, ether solvents, such as tetrahydrofuran, aromatic hydrocarbon solvents, such as toluene and xylene, ketone solvents, such as acetone and methyl ethyl ketone, and ester solvents, such as ethyl acetate, butyl acetate, and ethyl cellosolve acetate, are illustrated.

As the method for film formation from a solution or a melted state, coating methods, such as a spin coating method, a casting method, a microgravure coating method, a gravure coating method, a bar coating method, a roll coating method, a wire bar coating method, a dip coating method, a slit coating method, a capillary coating method, a spray coating method, a screen printing method, a flexographic printing method, an offset printing method, an ink jet printing method, and a nozzle coating method, can be used. Printing methods, such as a screen printing method, a flexographic printing method, an offset printing method, and an ink jet printing method, are preferred in that pattern formation and the separate application of many colors are easy.

As the polymer binder mixed, one not extremely inhibiting charge transport is preferred, and also, one not strongly absorbing visible light is suitably used. As the polymer binder, poly(N-vinylcarbazole), polyaniline and its derivatives, polythiophene and its derivatives, poly(p-phenylenevinylene) and its derivatives, poly(2,5-thienylenevinylene) and its derivatives, polycarbonate, polyacrylate, polymethyl acrylate, polymethyl methacrylate, polystyrene, polyvinyl chloride, polysiloxane, and the like are illustrated.

The optimal value of the film thickness of the electron transport layer is different depending on the material used, and the film thickness should be selected so that the driving voltage and the luminous efficiency are suitable values. However, at least a thickness such that no pinholes occur is necessary. If the thickness is too thick, the driving voltage of the device is high, so that too thick thickness is not preferred. Therefore, the film thickness of the electron transport layer is, for example, 1 nm to 1 µm, preferably 2 nm to 500 nm, and further preferably 5 nm to 200 nm.

Also, among the charge transport layers provided adjacent to the electrode, particularly, one having the function of improving the efficiency of charge injection from the electrode and having the effect of decreasing the driving voltage of the device may be generally called a charge injection layer (a hole injection layer or an electron injection layer).

Further, for an improvement in adhesion to the electrode, and an improvement in charge injection from the electrode, the charge injection layer or an insulating layer may be provided adjacent to the electrode. Also, for an improvement in the adhesion of the interface, the prevention of mixing, and the like, a thin buffer layer may be inserted at the interface of the charge transport layer and the light-emitting layer.

The order and number of layers laminated, and the thickness of each layer should be appropriately selected considering luminous efficiency and device life.

In the present invention, the polymer light-emitting device in which the charge injection layer (the electron injection layer or the hole injection layer) is provided includes a polymer light-emitting device in which the charge injection layer is provided adjacent to either one or both of the cathode and the anode.

For example, specifically, the following structures e) to p) are mentioned.
e) anode/charge injection layer/light-emitting layer/cathode
f) anode/light-emitting layer/charge injection layer/cathode
g) anode/charge injection layer/light-emitting layer/charge injection layer/cathode
h) anode/charge injection layer/hole transport layer/light-emitting layer/cathode
i) anode/hole transport layer/light-emitting layer/charge injection layer/cathode
j) anode/charge injection layer/hole transport layer/light-emitting layer/charge injection layer/cathode
k) anode/charge injection layer/light-emitting layer/charge transport layer/cathode
l) anode/light-emitting layer/electron transport layer/charge injection layer/cathode
m) anode/charge injection layer/light-emitting layer/electron transport layer/charge injection layer/cathode
n) anode/charge injection layer/hole transport layer/light-emitting layer/charge transport layer/cathode
o) anode/hole transport layer/light-emitting layer/electron transport layer/charge injection layer/cathode
p) anode/charge injection layer/hole transport layer/light-emitting layer/electron transport layer/charge injection layer/cathode

As specific examples of the charge injection layer, a layer comprising a conductive polymer, a layer provided between an anode and a hole transport layer and comprising a material having an ionization potential of an intermediate value between that of the anode material and that of the hole transport material included in the hole transport layer, a layer provided between a cathode and an electron transport layer and comprising a material having an electron affinity of an intermediate value between that of the cathode material and that of the electron transport material included in the electron transport layer, and the like are illustrated.

When the above charge injection layer is a layer comprising a conductive polymer, the conductivity of the conductive polymer is preferably 10⁻⁵ S/cm or more and 10³ S/cm or less, and to decrease the leak current between light-emitting pixels, the conductivity of the conductive polymer is more preferably 10⁻⁵ S/cm or more and 10² S/cm or less, and further preferably 10⁻⁵ S/cm or more and 10¹ S/cm or less. Usually, to provide the conductive polymer having a conductivity of 10⁻⁵ S/cm or more and 10³ S/cm or less, the conductive polymer is doped with a suitable amount of ions.

The type of doping ions is anions for the hole injection layer and cations for the electron injection layer. Examples of anions include polystyrenesulfonic acid ions, alkylbenzenesulfonic acid ions, and camphorsulfonic acid ions. Examples of cations include lithium ions, sodium ions, potassium ions, and tetrabutylammonium ions.

The film thickness of the charge injection layer is, for example, 1 nm to 100 nm, and preferably 2 nm to 50 nm.

The material used for the charge injection layer should be appropriately selected in relationship with the materials of the electrode and the adjacent layer, and conductive polymers, such as polyaniline and its derivatives, polythiophene and its derivatives, polypyrrole and its derivatives, polyphenylenevinylene and its derivatives, polythienylenevinylene and its derivatives, polyquinoline and its derivatives, polyquinoxaline and its derivatives, and a polymer comprising an aromatic amine structure in the main chain or the side chain, metal phthalocyanine (copper phthalocyanine and the like), carbon, and the like are illustrated.

The insulating layer has the function of facilitating charge injection. The average thickness of this insulating layer is usually 0.1 to 20 nm, preferably 0.5 to 10 nm, and more preferably 1 to 5 nm. The material of the insulating layer includes metal fluoride, metal oxide, and an organic insulating material. The polymer light-emitting device in which the insulating layer is provided includes a polymer light-emitting device in which the insulating layer is provided adjacent to either one or both of the cathode and the anode.

Specifically, for example, the following structures q) to ab) are mentioned.
q) anode/insulating layer/light-emitting layer/cathode
r) anode/light-emitting layer/insulating layer/cathode
s) anode/insulating layer/light-emitting layer/insulating layer/cathode
t) anode/insulating layer/hole transport layer/light-emitting layer/cathode
u) anode/hole transport layer/light-emitting layer/insulating layer/cathode
v) anode/insulating layer/hole transport layer/light-emitting layer/insulating layer/cathode
w) anode/insulating layer/light-emitting layer/electron transport layer/cathode
x) anode/light-emitting layer/electron transport layer/insulating layer/cathode
y) anode/insulating layer/light-emitting layer/electron transport layer/insulating layer/cathode
z) anode/insulating layer/hole transport layer/light-emitting layer/electron transport layer/cathode
aa) anode/hole transport layer/light-emitting layer/electron transport layer/insulating layer/cathode
ab) anode/insulating layer/hole transport layer/light-emitting layer/electron transport layer/insulating layer/cathode

The substrate forming the polymer light-emitting device of the present invention should be one that does not change in forming the electrode and the layer of an organic substance. For example, substrates of glass, plastic, a polymer film, silicon, and the like are illustrated. For an opaque substrate, the electrode opposite to the electrode closer to the substrate is preferably transparent or semitransparent.

In the present invention, usually, at least one of electrodes of an anode and a cathode is transparent or semitransparent, and preferably, the anode side is transparent or semitransparent.

As the material of the anode, a conductive metal oxide film, a semitransparent metal thin film, and the like are used. Specifically, a film made by conductive glass of indium oxide, zinc oxide, tin oxide, and composites thereof, indium tin oxide (ITO) and indium zinc oxide, and the like (NESA and the like), as well as gold, platinum, silver, copper, and the like are used, and ITO, indium zinc oxide, and tin oxide are preferred. The fabrication method includes a vacuum deposition method, a sputtering method, an ion plating method, and a plating method. Also, as the anode, an organic transparent conductive film of polyaniline and its derivatives, polythiophene and its derivatives, and the like may be used.

The film thickness of the anode can be appropriately selected considering light transmissiveness, and conductivity, and is, for example, 10 nm to 10 µm, preferably 20 nm to 1 µm, and further preferably 50 nm to 500 nm.

Also, to facilitate charge injection, a layer of a phthalocyanine derivative, a conductive polymer, carbon, or the like, or a layer of metal oxide, metal fluoride, an organic insulating material, or the like may be provided on the anode.

As the material of the cathode, a material having a small work function is preferred. For example, metals, such as lithium, sodium, potassium, rubidium, cesium, beryllium, magnesium, calcium, strontium, barium, aluminum, scandium, vanadium, zinc, yttrium, indium, cerium, samarium, europium, terbium, and ytterbium, and alloys of two or more of these, or alloys of one or more of these and one or more of gold, silver, platinum, copper, manganese, titanium, cobalt, nickel, tungsten, and tin, and graphite or intercalated graphite, and the like are used. Examples of the alloys include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy. The cathode may be a laminated structure of two or more layers.

The film thickness of the cathode can be appropriately selected considering conductivity and durability, and is, for example, 10 nm to 10 µm, preferably 20 nm to 1 µm, and further preferably 50 nm to 500 nm.

As the method for fabricating the cathode, a vacuum deposition method, a sputtering method, and also a lamination method in which a metal thin film is subjected to thermocompression, and the like are used. Also, a layer of a conductive polymer, or a layer of metal oxide, metal fluoride, an organic insulating material, or the like may be provided between the cathode and the organic layer, and after the fabrication of the cathode, a protective layer for protecting the polymer light-emitting device may be mounted. To use the polymer light-emitting device stably for a long period, preferably, a protective layer and/or a protective cover are mounted to externally protect the device.

As the protective layer, a resin, metal oxide, metal fluoride, metal boride, and the like can be used. Also, as the protective cover, a glass plate, a plastic plate having a surface subjected to low water permeability treatment, and the like can be used, and a method in which the cover is bonded to the device substrate with a thermosetting resin or a light curing resin for enclosure is suitably used. When a spacer is used to maintain space, it is easy to prevent the device from being damaged. When the space is filled with an inert gas, such as nitrogen and argon, cathode oxidation can be prevented. Further, by placing a drying agent, such as barium oxide, in the space, it is easy to suppress that moisture adsorbed in the manufacturing process damages the device. Preferably, any one or more measures of these are taken.

The polymer light-emitting device of the present invention can be used in displays, such as a planar light source, a segment display, a dot matrix display, and a liquid crystal display (for example, a backlight), and the like.

To obtain planar emission using the polymer light-emitting device of the present invention, a planar anode and cathode should be located to overlap each other. Also, to obtain patterned emission, there are a method in which a mask in which a patterned window is provided is placed on a surface of the planar light-emitting device, a method in which the organic layer in the non-light-emitting part is formed extremely thick for substantial non-emission, and a method in which either one of an anode or a cathode, or both are formed in a pattern. By forming a pattern by any of these methods and locating several electrodes to be independently turned ON/OFF, a segment type display device which can display numerals, letters, simple symbols, and the like is obtained. Further, to provide a dot matrix device, an anode and a cathode both should be formed in stripes and located orthogonal to each other. By a method in which a plurality of types of polymer compounds having different emission colors are separately applied, and a method using a color filter or a fluorescence conversion filter, partial color display and multicolor display are possible. For the dot matrix device, passive drive is also possible, and active drive may be performed in combination with TFT and the like. These display devices can be used as the displays of a computer, a television, a portable terminal, a cellular phone, car navigation, a video camera viewfinder, and the like.

Further, the planar light-emitting device is of a self-light-emitting thin type and can be suitably used as a planar light source for the backlight of a liquid crystal display, or a planar light source for illumination. Also, when a flexible substrate is used, the light-emitting device can also be used as a curved light source and display.

### EXAMPLES

Examples and comparative examples are illustrated below to describe the present invention in more detail, however, the present invention is not limited to these examples.

### (Example 1) Synthesis of Polymer Compound <P-1>

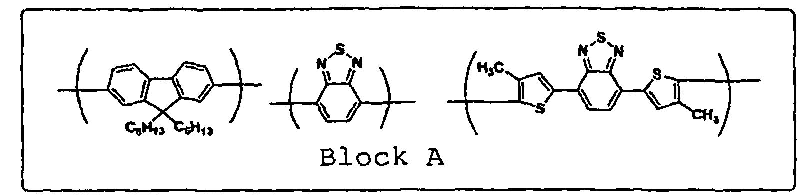

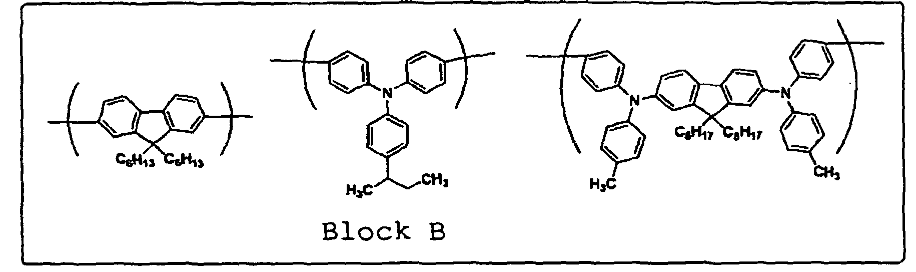

In an inert atmosphere, 2,7-bis(1,3,2-dioxaborolane-2-yl)-9,9-dihexylfluorene (0.69 g), 4,7-dibromo-2,1,3-benzothiadiazole (0.40 g), 4,7-bis(5-bromo-4-methyl-2-thienyl)-2,1,3-benzothiadiazole (0.11 g), dichlorobis(triphenylphosphine)palladium (3.3 mg), Aliquat 336 (0.20 g, manufactured by Aldrich), and toluene (16 mL) were mixed and heated to 105°C. A 2M Na₂CO₃ aqueous solution (5 mL) was dropped into this reaction solution, and the mixture was refluxed for 4 hours. The weight average molecular weight based on polystyrene standards, at this time, was 2.4 × 10⁴. After cooling to 70°C, 2,7-bis(1,3,2-dioxaborolane-2-yl)-9,9-dihexylfluorene (1.46 g), 2,7-dibromo-9,9-dihexylfluorene (0.66 g), bis(4-bromophenyl)-(4-secondary butylphenyl)-amine (0.52 g), N,N'-bis-(4-bromophenyl)-N,N'-di-p-toluyl-9,9-dioctylfluorene-2,7-diamine (0.41 g), dichlorobis(triphenylphosphine)palladium (6.2 mg), Aliquat 336 (0.38g, manufactured by Aldrich), and toluene (30 mL) were mixed with the reaction solution and heated to 105°C. A 2M Na₂CO₃ aqueous solution (8 mL) was dropped into this reaction solution, and the mixture was refluxed for 7 hours. After reaction, phenyl boric acid (0.05 g) was added, and the mixture was further refluxed for 2 hours. Then, a sodium diethyldithiacarbamate aqueous solution was added, and the mixture was stirred at 80°C for 2 hours. After cooling, the solution was washed with water (100 mL) three times, with a 3 wt% acetic acid aqueous solution (100 mL) three times, and with water (100 mL) three times, and passed through an alumina column and a silica gel column for purification. The obtained toluene solution was dropped into methanol (1.5 L), and the mixture was stirred for 1 hour, then, the obtained solid was filtered and dried. The yield of the obtained polymer compound <P-1> was 2.45 g.
The polymer compound <P-1> had a polystyrene equivalent weight average molecular weight of 2.9 × 10⁵ and a polystyrene equivalent number average molecular weight of 9.5 × 10⁴.

4,7-dibromo-2,1,3-benzothiadiazole was synthesized by a method described in US3577427. 4,7-bis(5-bromo-2-thienyl)-2,1,3-benzothiadiazole and 4,7-bis(5-bromo-4-methyl-2-thienyl)-2,1,3-benzothiadiazole were synthesized by a method described in WO00/046321. Bis(4-bromophenyl)-(4-secondary butylphenyl)-amine was synthesized by a method described in WO02/045184. N,N'-bis-(4-bromophenyl)-N,N'-di-p-toluyl-9,9-dioctylfluorene-2,7-diamine was synthesized by a method described in WO2005/049548.

### (Comparative Example 1) Synthesis of Polymer Compound <P-2>

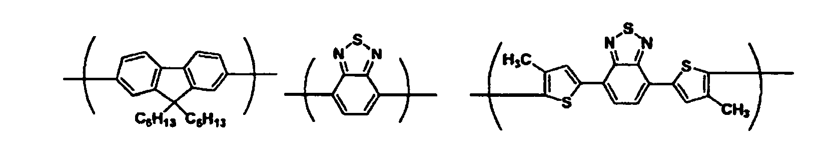

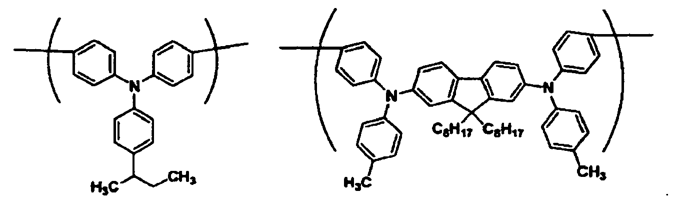

In an inert atmosphere, 2,7-bis(1,3,2-dioxaborolane-2-yl)-9,9-dihexylfluorene (0.69 g), 4,7-dibromo-2,1,3-benzothiadiazole (0.35 g), 4,7-bis(5-bromo-4-methyl-2-thienyl)-2,1,3-benzothiadiazole (0.10 g), 2,7-dibromo-9,9-dihexylfluorene (0.59 g), bis(4-bromophenyl)-(4-secondary butylphenyl)-amine (0.46 g), N,N'-bis-(4-bromophenyl)-N,N'-di-p-toluyl-9,9-dioctylfluorene-2,7-diamine (0.36 g), dichlorobis(triphenylphosphine)palladium (8.4 mg), Aliquat 336 (0.52g, manufactured by Aldrich), and toluene (40 mL) were mixed and heated to 105°C. A 2M Na₂CO₃ aqueous solution (11 mL) was dropped into this reaction solution, and the mixture was refluxed for 7 hours. After reaction, phenyl boric acid (0.05 g) was added, and the mixture was further refluxed for 2 hours. Then, a sodium diethyldithiacarbamate aqueous solution was added, and the mixture was stirred at 80°C for 2 hours. After cooling, the solution was washed with water (100 mL) three times, with a 3 wt% acetic acid aqueous solution (100 mL) three times, and with water (100 mL) three times, and passed through an alumina column and a silica gel column for purification. The obtained toluene solution was dropped into methanol (1.5 L), and the mixture was stirred for 1 hour, then, the obtained solid was filtered and dried. The yield of the obtained polymer compound <P-2> was 1.83 g.
The polymer compound <P-2> had a polystyrene equivalent weight average molecular weight of 2.6 × 10⁵ and a polystyrene equivalent number average molecular weight of 9.8 × 10⁴.

(Synthesis Example 1) Synthesis of Polymer Compound <P-3>

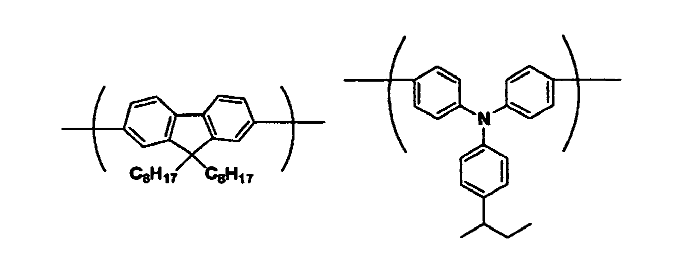

In an inert atmosphere, 2,7-bis(1,3,2-dioxaborolane-2-yl)-9,9-dioctylfluorene (5.20 g), bis(4-bromophenyl)-(4-secondary butylphenyl)-amine (0.14 g), palladium acetate (2.2 mg), tri(2-methylphenyl)phosphine (15.1 mg), Aliquat 336 (0.91 g, manufactured by Aldrich), and toluene (70 mL) were mixed and heated to 105°C. A 2M Na₂CO₃ aqueous solution (19 ml) was dropped into this reaction solution, and the mixture was refluxed for 4 hours. After reaction, phenyl boric acid (121 mg) was added, and the mixture was further refluxed for 3 hours. Then, a sodium diethyldithiacarbamate aqueous solution was added, and the mixture was stirred at 80°C for 4 hours. After cooling, the solution was washed with water (60 ml) three times, with a 3 wt% acetic acid aqueous solution (60 ml) three times, and with water (60 ml) three times, and passed through an alumina column and a silica gel column for purification. The obtained toluene solution was dropped into methanol (3 L), and the mixture was stirred for 3 hours, then, the obtained solid was filtered and dried. The yield of the obtained polymer compound <P-3> was 5.25 g.
The polymer compound <P-3> had a polystyrene equivalent weight average molecular weight of 2.6 × 10⁵ and a polystyrene equivalent number average molecular weight of 1.2 × 10⁵.

(Comparative Example 2) Synthesis of Polymer Compound <P-4>

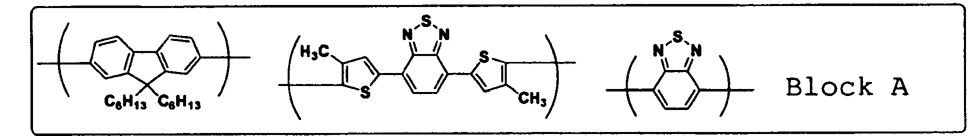

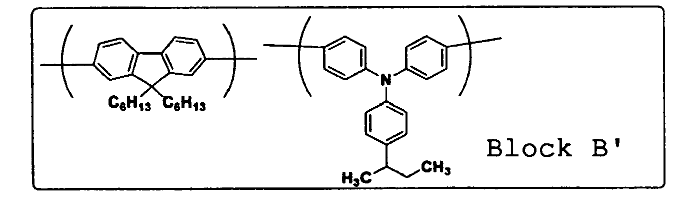

In an inert atmosphere, 2,7-bis(1,3,2-dioxaborolane-2-yl)-9,9-dihexylfluorene (0.77 g), 4,7-dibromo-2,1,3-benzothiadiazole (0.44 g), 4,7-bis(5-bromo-4-methyl-2-thienyl)-2,1,3-benzothiadiazole (0.15 g), dichlorobis(triphenylphosphine)palladium (3.8 mg), Aliquat 336 (0.25 g, manufactured by Aldrich), and toluene (25 mL) were mixed and heated to 105°C. A 2M Na₂CO₃ aqueous solution (5 mL) was dropped into this reaction solution, and the mixture was refluxed for 2 hours. The weight average molecular weight based on polystyrene standards, at this time, was 7.2 × 10³. After cooling to 70°C, 2,7-bis(1,3,2-dioxaborolane-2-yl)-9,9-dihexylfluorene (2.09 g), 2,7-dibromo-9,9-dihexylfluorene (1.18 g), bis(4-bromophenyl)-(4-secondary butylphenyl)-amine (0.83 g), dichlorobis(triphenylphosphine)palladium (8.8 mg), Aliquat 336 (0.55 g, manufactured by Aldrich), and toluene (45 mL) were mixed with the reaction solution and heated to 105°C. A 2M Na₂CO₃ aqueous solution (11.4 mL) was dropped into this reaction solution, and the mixture was refluxed for 1 hour. After reaction, phenyl boric acid (0.07 g) was added, and the mixture was further refluxed for 2 hours. Then, a sodium diethyldithiacarbamate aqueous solution was added, and the mixture was stirred at 80°C for 2 hours. After cooling, the solution was washed with water (80 mL) two times, with a 3 wt% acetic acid aqueous solution (80 mL) two times, and with water (80 mL) two times, and passed through an alumina column and a silica gel column for purification. The obtained toluene solution was dropped into methanol (1.0 L), and the mixture was stirred for 1 hour, then, the obtained solid was filtered and dried. The yield of the obtained polymer compound <P-4> was 2.76 g.
The polymer compound <P-4> had a polystyrene equivalent number average molecular weight of 8.8 × 10⁴ and a polystyrene equivalent weight average molecular weight of 1.9 × 10⁵.

### [Fabrication and Evaluation of Electroluminescent (EL) Device]

### (Example 2)

### - Fabrication of Light-Emitting Device

A suspension of poly(3,4)ethylenedioxythiophene/polystyrenesulfonic acid (manufactured by Bayer, trade name: BaytronP) was placed on a glass substrate, on which an ITO film having a thickness of 150 nm was provided by a sputtering method, formed into a film having a thickness of about 65 nm by a spin coating method, and dried on a hot plate at 200°C for 15 minutes. Next, the polymer compound <P-3> was dissolved in mixed xylene at a concentration of 1.5 wt%. The obtained xylene solution was placed on the film of BaytronP, formed into a film by the spin coating method, and then dried in a nitrogen atmosphere with an oxygen concentration of 10 ppm or less and a moisture concentration of 10 ppm or less (on a weight basis) at 200°C for 1 hour. After drying, the soluble portion was rinsed using mixed xylene in the air to provide the film of the polymer compound <P-3> having a thickness of about 10 nm. Next, the polymer compound <P-1> was dissolved in mixed xylene at a concentration of 1.5 wt%. The obtained xylene solution was placed on the film of the polymer compound <P-3>, formed into a film having a thickness of about 100 nm by the spin coating method, and then dried in a nitrogen atmosphere with an oxygen concentration of 10 ppm or less and a moisture concentration of 10 ppm or less (on a weight basis) at 130°C for 1 hour. After the pressure was reduced to 1.0 × 10⁻⁴ Pa or less, as the cathode, about 5 nm of barium was vapor deposited on the film of the polymer compound <P-1>, and then, about 80 nm of aluminum was vapor deposited on the layer of barium. After vapor deposition, sealing was performed using a glass substrate to fabricate a polymer light-emitting device. The configuration of the device was as follows:

ITO/BaytronP (about 70 nm)/polymer compound <P-3> (10 nm)/polymer compound <P-1> (about 100 nm)/Ba/Al

### - Performance of EL Device

When a voltage of 10.0 V was applied to the obtained polymer light-emitting device, the polymer light-emitting device emitted fluorescence in which the peak top of emission wavelength was 645 nm. At the time, the luminance was 5315 cd/m², and the luminous efficiency was 1.5 cd/A.

### (Comparative Example 3)

### - Fabrication of Light-Emitting Device

A polymer light-emitting device was fabricated as in Example 2, except that the polymer compound <P-2> was used instead of the polymer compound <P-1> in Example 2. The configuration of the device was as follows:

ITO/BaytronP (about 70 nm)/polymer compound <P-3> (10 nm)/polymer compound <P-2> (about 100 nm)/Ba/Al

### - Performance of EL Device

When a voltage of 10.0 V was applied to the obtained polymer light-emitting device, the polymer light-emitting device emitted fluorescence in which the peak top of emission wavelength was 645 nm. At the time, the luminance was 3062 cd/m², and the luminous efficiency was 1.3 cd/A.

### INDUSTRIAL APPLICABILITY

As described above, the present invention can provide a block copolymer with which a light-emitting device that can provide high-luminance emission can be manufactured when it is used as a material of a light-emitting device, and a composition, a liquid composition, a light-emitting thin film, and a polymer light-emitting device using the same.

The block copolymer of the present invention is useful as the light-emitting material, hole transport material, and electron transport material of a light-emitting device, and a light-emitting device comprising the block copolymer of the present invention can be used for a curved light source and a light source on a plane for the backlight of a liquid crystal display or for illumination, and the like.

## Claims

1. A block copolymer **characterized by** comprising:
a block (A) comprising a repeating unit represented by the following formula (I), and a repeating unit represented by the following formula (II); and
a block (B) comprising a repeating unit represented by the following formula (II), a repeating unit represented by the following formula (III), and a repeating unit represented by the following formula (IV),
-Ar₁- (I)
wherein Ar₁ represents a divalent heterocyclic group containing a five-membered ring,
-Ar₂- (II)
wherein Ar₂ represents an arylene group, 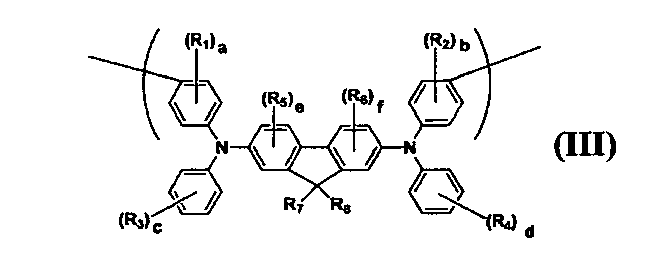 wherein R₁, R₂, R₃, R₄, R₅, and R₆ independently represent a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group,
a and b independently represent an integer of 0 to 4,
c and d independently represent an integer of 0 to 5, and
e and f independently represent an integer of 0 to 3,
provided that when a plurality of at least one of R₁, R₂, R₃, R₄, R₅, and R₆ are present, the plurality of atoms or groups may be the same or different,
R₇ and R₈ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and 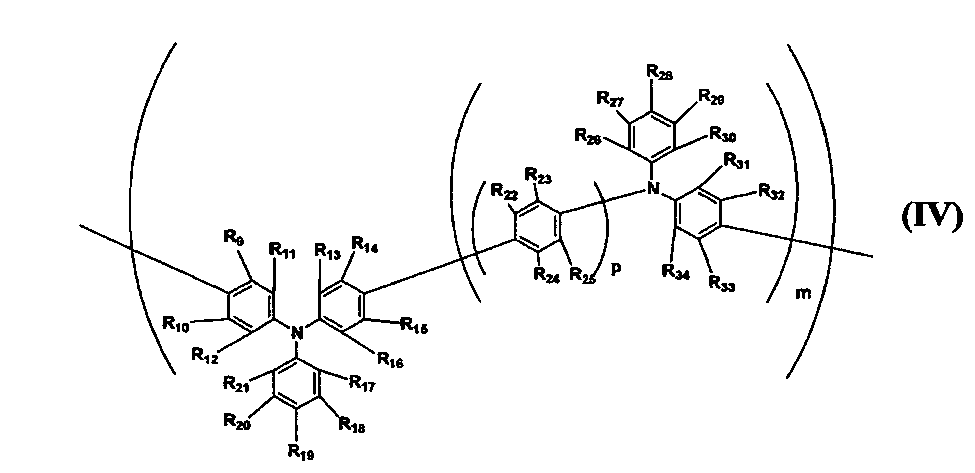 wherein R₉ to R₃₄ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and
m and p independently represent 0 or 1,
provided that at least one of combinations of R₁₁ and R₁₃, R₁₂ and R₂₁, R₂₅ and R₃₄, and R₃₀ and R₃₁ may be taken together to form a single bond or a divalent group represented by the formula: -O- or the formula: - S-, instead of representing the atom or group.

2. The block copolymer according to claim 1,
wherein in the formula (I), Ar₁ is a group represented by the following formula (V): 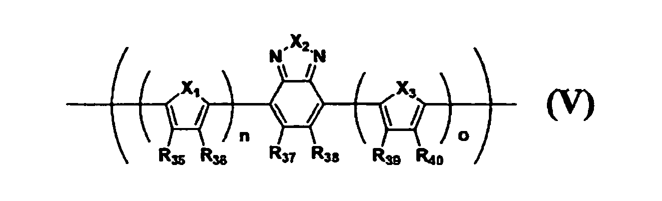 wherein R₃₅, R₃₆, R₃₇, R₃₈, R₃₉, and R₄₀ independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group,
n and o independently represent an integer of 0 to 3, and
X₁, X₂, and X₃ independently represent an oxygen atom or a sulfur atom,
provided that when a plurality of at least one of R₃₅, R₃₆, R₃₉, R₄₀, X₁, and X₃ are present, the plurality of atoms or groups may be the same or different.

3. The block copolymer according to claim 2, wherein in the formula (V), X₁, X₂, and X₃ are a sulfur atom.

4. The block copolymer according to any one of claims 1 to 3, wherein in the formula (II), Ar₂ is a group represented by the following formula (VI): 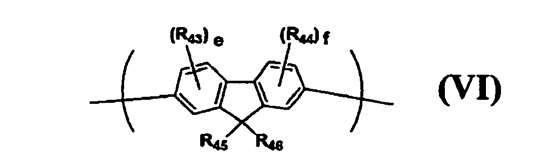 wherein R₄₃ and R₄₄ independently represent a halogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group, and
e and f independently represent an integer of 0 to 3,
provided that when a plurality of at least one of R₄₃ and R₄₄ are present, the plurality of atoms or groups may be the same or different, and
R₄₅ and R₄₆ independently represent a hydrogen atom, a halogen atom, an alkyl group, an alkoxy group, an alkylthio group, an aryl group, an a*ryloxy group, an arylthio group, an arylalkyl group, an arylalkoxy group, an arylalkylthio group, an arylalkenyl group, an arylalkynyl group, a monovalent heterocyclic group, a heterocyclic thio group, an amino group, a silyl group, an acyl group, an acyloxy group, an imine residue, an amide group, an acid imide group, a carboxyl group, a cyano group, or a nitro group.

5. The block copolymer according to any one of claims 1 to 4, wherein in the formula (IV), p and m are 0.

6. The block copolymer according to any one of claims 1 to 5, having a polystyrene equivalent weight average molecular weight of 1 × 10³ to 1 × 10⁷.

7. The block copolymer according to any one of claims 1 to 6, wherein the block (A) has a polystyrene equivalent weight average molecular weight of 1 × 10³ to 1 × 10⁵.

8. The block copolymer according to any one of claims 1 to 7, wherein [A]/[B] is 0.1 or more and 10 or less, wherein [A] represents a mole fraction of the block (A) with respect to a total of the blocks (A) and (B), and [B] represents a mole fraction of the block (B) with respect to the total of the blocks (A) and (B).

9. A composition **characterized by** comprising:
the block copolymer according to any one of claims 1 to 8; and
a solvent, a light-emitting material other than the block copolymer, a hole transport material other than the block copolymer, or an electron transport material other than the block copolymer, or a combination of two or more thereof.

10. The composition according to claim 9, containing at least two types of the block copolymers.

11. A light-emitting thin film **characterized by** comprising the block copolymer according to any one of claims 1 to 8.

12. The light-emitting thin film according to claim 11, further containing a light-emitting material other than the block copolymer, a hole transport material other than the block copolymer, or an electron transport material other than the block copolymer, or a combination of two or more thereof.

13. A polymer light-emitting device **characterized by** comprising:
an anode;
a cathode; and
an organic layer comprising the block copolymer according to any one of claims 1 to 8 and provided between the anode and the cathode.

14. The polymer light-emitting device according to claim 13, wherein the organic layer further comprises a light-emitting material other than the block copolymer, a hole transport material other than the block copolymer, or an electron transport material other than the block copolymer, or a combination of two or more thereof.

15. The polymer light-emitting device according to claim 13 or 14, wherein the organic layer is a light-emitting layer.

## Patentansprüche

1. Ein Blockcopolymer, **dadurch gekennzeichnet, dass** es umfasst:
einen Block (A), der eine durch die folgende Formel (I) dargestellte wiederkehrende Einheit und eine durch die folgende Formel (II) dargestellte wiederkehrende Einheit umfasst; und
einen Block (B), der eine durch die folgende Formel (II) dargestellte wiederkehrende Einheit, eine durch die folgende Formel (III) dargestellte wiederkehrende Einheit und
eine durch die folgende Formel (IV) dargestellte wiederkehrende Einheit umfasst,
- Ar₁- (I)
wobei Ar₁ einen zweiwertigen heterocyclischen Rest, der einen fünfgliedrigen Ring enthält, bedeutet,
- Ar₂- (II)
wobei Ar₂ einen Arylenrest bedeutet, 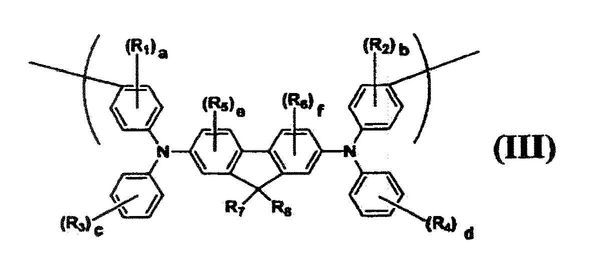
wobei R₁, R₂, R₃, R₄, R₅ und R₆ unabhängig ein Halogenatom, einen Alkylrest, einen Alkoxyrest, einen Alkylthiorest, einen Arylrest, einen Aryloxyrest, einen Arylthiorest,
einen Arylalkylrest, einen Arylalkoxyrest, einen Arylalkylthiorest, einen Arylalkenylrest,
einen Arylalkinylrest, einen einwertigen heterocyclischen Rest, einen heterocyclischen Thiorest, eine Aminogruppe, eine Silylgruppe, einen Acylrest, einen Acyloxyrest, einen Iminrest, einen Amidrest, einen Säureimidrest, eine Carboxylgruppe, eine Cyanogruppe oder eine Nitrogruppe bedeuten,
a und b unabhängig eine ganze Zahl von 0 bis 4 bedeuten,
c und d unabhängig eine ganze Zahl von 0 bis 5 bedeuten und
e und f unabhängig eine ganze Zahl von 0 bis 3 bedeuten,
mit der Maßgabe, dass, wenn eine Vielzahl von mindestens einem von R₁, R₂, R₃, R₄, R₅ und R₆ vorhanden sind, die Vielzahl von Atomen oder Resten gleich oder verschieden sein können,
R₇ und R₈ unabhängig ein Wasserstoffatom, ein Halogenatom, einen Alkylrest, einen Alkoxyrest, einen Alkylthiorest, einen Arylrest, einen Aryloxyrest, einen Arylthiorest,
einen Arylalkylrest, einen Arylalkoxyrest, einen Arylalkylthiorest, einen Arylalkenylrest,
einen Arylalkinylrest, einen einwertigen heterocyclischen Rest, einen heterocyclischen Thiorest, eine Aminogruppe, eine Silylgruppe, einen Acylrest, einen Acyloxyrest, einen Iminrest, einen Amidrest, einen Säureimidrest, eine Carboxylgruppe, eine Cyanogruppe
oder eine Nitrogruppe bedeuten und 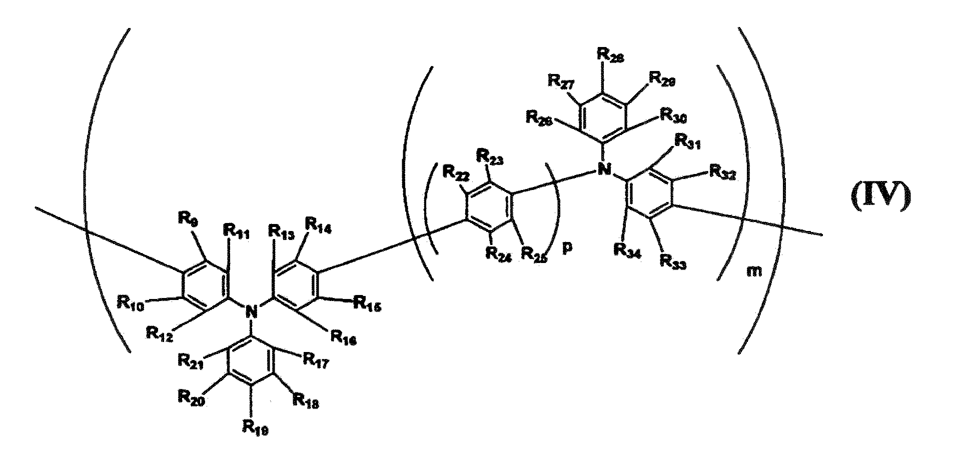
wobei R₉ bis R₃₄ unabhängig ein Wasserstoffatom, ein Halogenatom, einen Alkylrest,
einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Arylalkenylrest, einen Arylalkinylrest, einen einwertigen heterocyclischen Rest, einen heterocyclischen Thiorest, eine Aminogruppe, eine Silylgruppe, einen Acylrest, einen Acyloxyrest, einen Iminrest, einen Amidrest, einen Säureimidrest, eine Carboxylgruppe,
eine Cyanogruppe oder eine Nitrogruppe bedeuten und
m und p unabhängig 0 oder 1 bedeuten,
mit der Maßgabe, dass mindestens eine der Kombinationen von R₁₁ und R₁₃, R₁₂ und R₂₁,
R₂₅ und R₃₄, und R₃₀ und R₃₁ zusammengenommen werden kann, um eine Einfachbindung oder einen zweiwertigen Rest, dargestellt durch die Formel: -O- oder die Formel -S-, zu bilden, anstatt das Atom oder den Rest darzustellen.

2. Das Blockcopolymer nach Anspruch 1, wobei in der Formel (I) Ar₁ ein durch die folgende Formel (V) dargestellter Rest ist: 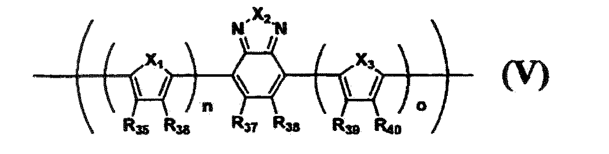
wobei R₃₅, R₃₆, R₃₇, R₃₈, R₃₉ und R₄₀ unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Arylalkenylrest, einen Arylalkinylrest, einen einwertigen heterocyclischen Rest, einen heterocyclischen Thiorest, eine Aminogruppe, eine Silylgruppe, einen Acylrest, einen Acyloxyrest, einen Iminrest, einen Amidrest, einen Säureimidrest, eine Carboxylgruppe, eine Cyanogruppe oder eine Nitrogruppe bedeuten,
n und o unabhängig eine ganze Zahl von 0 bis 3 bedeuten und
X₁, X₂ und X₃ unabhängig ein Sauerstoffatom oder ein Schwefelatom bedeuten,
mit der Maßgabe, dass, wenn eine Vielzahl von mindestens einem von R₃₅, R₃₆, R₃₉, R₄₀, X₁ und X₃ vorhanden sind, die Vielzahl von Atomen oder Resten gleich oder verschieden sein können.

3. Das Blockcopolymer nach Anspruch 2, wobei in der Formel (V) X₁, X₂ und X₃ ein Schwefelatom sind.

4. Das Blockcopolymer nach einem der Ansprüche 1 bis 3, wobei in der Formel (II) Δr₂ ein durch die folgende Formel (VI) dargestellter Rest ist: 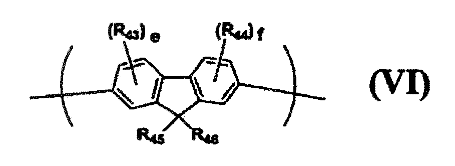
wobei R₄₃ und R₄₄ unabhängig ein Halogenatom, einen Alkylrest, einen Alkoxyrest,
einen Alkylthiorest, einen Arylrest, einen Aryloxyrest, einen Arylthiorest, einen Arylalkylrest, einen Arylalkoxyrest, einen Arylalkylthiorest, einen Arylalkenylrest, einen Arylalkinylrest, einen einwertigen heterocyclischen Rest, einen heterocyclischen Thiorest, eine Aminogruppe, eine Silylgruppe, einen Acylrest, einen Acyloxyrest, einen Iminrest, einen Amidrest, einen Säureimidrest, eine Carboxylgruppe, eine Cyanogruppe oder eine Nitrogruppe bedeuten und
e und f unabhängig einen ganze Zahl von 0 bis 3 bedeuten,
mit der Maßgabe, dass, wenn eine Vielzahl von mindestens einem von R₄₃ und R₄₄ vorhanden sind, die Vielzahl von Atomen oder Resten gleich oder verschieden sein können, und
R₄₅ und R₄₆ unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Arylalkenylrest, einen Arylalkinylrest, einen einwertigen heterocyclischen Rest, einen heterocyclischen Thiorest, eine Aminogruppe, eine Silylgruppe, einen Acylrest, einen Acyloxyrest, einen Iminrest, einen Amidrest, einen Säureimidrest, eine Carboxylgruppe, eine Cyanogruppe oder eine Nitrogruppe bedeuten.

5. Das Blockcopolymer nach einem der Ansprüche 1 bis 4, wobei in der Formel (IV) p und m 0 sind.

6. Das Blockcopolymer nach einem der Ansprüche 1 bis 5 mit einem Polystyroläquivalenten Gewichtsmittel des Molekulargewichts von 1 x 10³ bis 1 x 10⁷.

7. Das Blockcopolymer nach einem der Ansprüche 1 bis 6, wobei der Block (A) ein Polystyrol-äquivalentes Gewichtsmittel des Molekulargewichts von 1 x 10³ bis 1 x 10⁵ aufweist.

8. Das Blockcopolymer nach einem der Ansprüche 1 bis 7, wobei [A]/[B] 0,1 oder mehr und 10 oder weniger beträgt, wobei [A] eine Molfraktion des Blocks (A), bezogen auf eine Gesamtheit der Blöcke (A) und (B), bedeutet und [B] eine Molfraktion des Blocks (B), bezogen auf die Gesamtheit der Blöcke (A) und (B), bedeutet.

9. Eine Zusammensetzung, **dadurch gekennzeichnet, dass** sie umfasst:
das Blockcopolymer nach einem der Ansprüche 1 bis 8; und
ein Lösungsmittel, ein von dem Blockcopolymer verschiedenes lichtemittierendes Material, ein von dem Blockcopolymer verschiedenes lochtransportierendes Material oder ein von dem Blockcopolymer verschiedenes elektronentransportierendes Material oder eine Kombination von zwei oder mehr davon.

10. Die Zusammensetzung nach Anspruch 9, welche mindestens zwei Arten der Blockcopolymere enthält.

11. Eine lichtemittierende Dünnfolie, **dadurch gekennzeichnet, dass** sie das Blockcopolymer nach einem der Ansprüche 1 bis 8 umfasst.

12. Die lichtemittierende Dünnfolie nach Anspruch 11, welche weiter ein von dem Blockcopolymer verschiedenes lichtemittierendes Material, ein von dem Blockcopolymer verschiedenes lochtransportierendes Material oder ein von dem Blockcopolymer verschiedenes elektronentransportierendes Material oder eine Kombination von zwei oder mehr davon enthält.

13. Eine polymere lichtemittierende Vorrichtung, **dadurch gekennzeichnet, dass** sie umfasst:
eine Anode;
eine Kathode; und
eine organische Schicht, die das Blockcopolymer nach einem der Ansprüche 1 bis 8 umfasst und zwischen der Anode und der Kathode bereitgestellt ist.

14. Die polymere lichtemittierende Vorrichtung nach Anspruch 13, wobei die organische Schicht weiter ein von dem Blockcopolymer verschiedenes lichtemittierendes Material, ein von dem Blockcopolymer verschiedenes lochtransportierendes Material oder ein von dem Blockcopolymer verschiedenes elektronentransportierendes Material oder eine Kombination von zwei oder mehr davon umfasst.

15. Die polymere lichtemittierende Vorrichtung nach Anspruch 13 oder 14, wobei die organische Schicht eine lichtemittierende Schicht ist.

## Revendications

1. Copolymère à blocs, **caractérisé en ce qu'**il comporte :
- un bloc (A) comportant un motif répété représenté par la formule (I) présentée ci-dessous et un motif répété représenté par la formule (II) présentée ci-dessous;
- et un bloc (B) comportant un motif répété représenté par la formule (II) présentée ci-dessous, un motif répété représenté par la formule (III) présentée ci-dessous et un motif répété représenté par la formule (IV) présentée ci-dessous ;
-Ar₁- (I)
dans laquelle formule Ar₁ représente un groupe hétérocyclique divalent comportant un cycle à 5 chaînons ;
—Ar₂— (II)
dans laquelle formule Ar₂ représente un groupe arènediyle ; 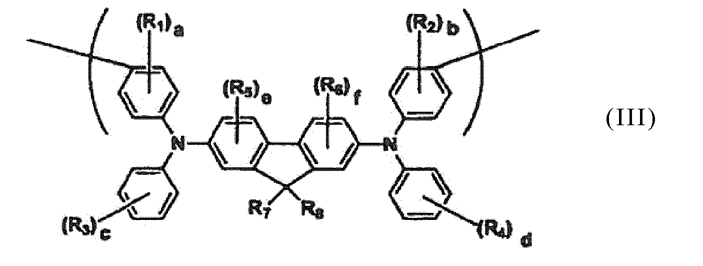 dans laquelle formule
R₁, R₂, R₃, R₄, R₅ et R₆ représentent chacun, indépendamment, un atome d'halogène, un groupe alkyle, un groupe alcoxy, un groupe alkylthio, un groupe aryle, un groupe aryloxy, un groupe arylthio, un groupe aryl-alkyle, un groupe aryl-alcoxy, un groupe aryl-alkylthio, un groupe aryl-alcényle, un groupe aryl-alcynyle, un groupe hétérocyclique monovalent, un groupe hétérocyclyl-thio, un groupe amino, un groupe silyle, un groupe acyle, un groupe acyl-oxy, un résidu de type imine, un groupe de type amide, un groupe de type imide acide, un groupe carboxyle, un groupe cyano ou un groupe nitro,
les indices a et b sont chacun, indépendamment, un nombre entier valant de 0 à 4,
les indices c et d sont chacun, indépendamment, un nombre entier valant de 0 à 5,
les indices e et f sont chacun, indépendamment, un nombre entier valant de 0 à 3,
étant entendu que, s'il y a plusieurs atomes ou groupes représentés par l'un au moins des symboles R₁, R₂, R₃, R₄, R₅ et R₆, ils peuvent être identiques ou différents,
R₇ et R₈ représentent chacun, indépendamment, un atome d'hydrogène, un atome d'halogène, un groupe alkyle, un groupe alcoxy, un groupe alkylthio, un groupe aryle, un groupe aryloxy, un groupe arylthio, un groupe aryl-alkyle, un groupe aryl-alcoxy, un groupe aryl-alkylthio, un groupe aryl-alcényle, un groupe aryl-alcynyle, un groupe hétérocyclique monovalent, un groupe hétérocyclyl-thio, un groupe amino, un groupe silyle, un groupe acyle, un groupe acyloxy, un résidu de type imine, un groupe de type amide, un groupe de type imide acide, un groupe carboxyle, un groupe cyano ou un groupe nitro ; 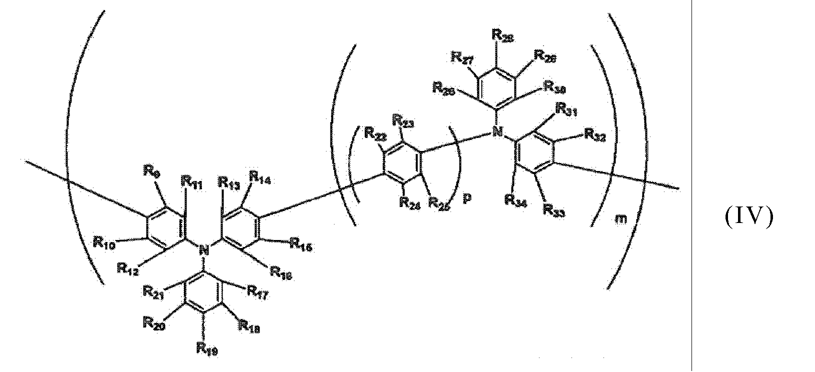 dans laquelle
les symboles R₉ à R₃₄ représentent chacun, indépendamment, un atome d'hydrogène, un atome d'halogène, un groupe alkyle, un groupe alcoxy, un groupe alkylthio, un groupe aryle, un groupe aryloxy, un groupe arylthio, un groupe aryl-alkyle, un groupe aryl-alcoxy, un groupe aryl-alkylthio, un groupe aryl-alcényle, un groupe aryl-alcynyle, un groupe hétérocyclique monovalent, un groupe hétérocyclyl-thio, un groupe amino, un groupe silyle, un groupe acyle, un groupe acyl-oxy, un résidu de type imine, un groupe de type amide, un groupe de type imide acide, un groupe carboxyle, un groupe cyano ou un groupe nitro,
et les indices m et p valent chacun, indépendamment, 0 ou 1,
étant entendu que les symboles d'au moins l'une des combinaisons R₁₁ et R₁₃, R₁₂ et R₂₁, R₂₅ et R₃₄, et R₃₀ et R₃₁, peuvent représenter conjointement une liaison simple ou un chaînon divalent symbolisé par -O- ou -S-, au lieu de représenter un atome ou un groupe.

2. Copolymère à blocs conforme à la revendication 1, pour lequel, dans la formule générale (I), Ar₁ représente un groupe de formule générale (V) suivante : 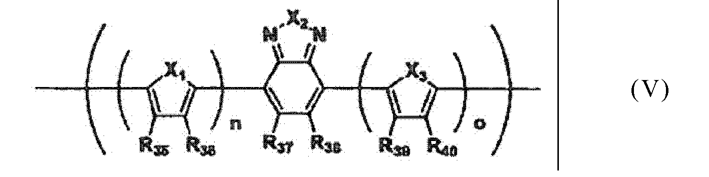
dans laquelle R₃₅, R₃₆, R₃₇, R₃₈, R₃₉ et R₄₀ représentent chacun, indépendamment, un atome d'hydrogène, un atome d'halogène, un groupe alkyle, un groupe alcoxy, un groupe alkylthio, un groupe aryle, un groupe aryloxy, un groupe arylthio, un groupe aryl-alkyle, un groupe aryl-alcoxy, un groupe aryl-alkylthio, un groupe aryl-alcényle, un groupe aryl-alcynyle, un groupe hétérocyclique monovalent, un groupe hétérocyclyl-thio, un groupe amino, un groupe silyle, un groupe acyle, un groupe acyloxy, un résidu de type imine, un groupe de type amide, un groupe de type imide acide, un groupe carboxyle, un groupe cyano ou un groupe nitro,
les indices n et o sont chacun, indépendamment, un nombre entier valant de 0 à 3,
et X₁, X₂ et X₃ représentent chacun, indépendamment, un atome d'oxygène ou un atome de soufre,
étant entendu que, s'il y a plusieurs atomes ou groupes représentés par l'un au moins des symboles R₃₅, R₃₆, R₃₉, R₄₀, X₁ et X₃, ils peuvent être identiques ou différents.

3. Copolymère à blocs conforme à la revendication 2, pour lequel, dans la formule (V), X₁, X₂ et X₃ représentent chacun un atome de soufre.

4. Copolymère à blocs conforme à l'une des revendications 1 à 3, pour lequel, dans la formule (II), Ar₂ représente un groupe de formule (VI) suivante : 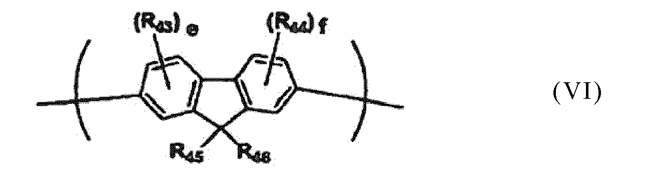 dans laquelle
R₄₃ et R₄₄ représentent chacun, indépendamment, un atome d'halogène, un groupe alkyle, un groupe alcoxy, un groupe alkylthio, un groupe aryle, un groupe aryloxy, un groupe arylthio, un groupe aryl-alkyle, un groupe aryl-alcoxy, un groupe aryl-alkylthio, un groupe aryl-alcényle, un groupe aryl-alcynyle, un groupe hétérocyclique monovalent, un groupe hétérocyclyl-thio, un groupe amino, un groupe silyle, un groupe acyle, un groupe acyloxy, un résidu de type imine, un groupe de type amide, un groupe de type imide acide, un groupe carboxyle, un groupe cyano ou un groupe nitro,
les indices e et f sont chacun, indépendamment, un nombre entier valant de 0 à 3,
étant entendu que, s'il y a plusieurs atomes ou groupes représentés par l'un au moins des symboles R₄₃ et R₄₄, ils peuvent être identiques ou différents,
et R₄₅ et R₄₆ représentent chacun, indépendamment, un atome d'hydrogène, un atome d'halogène, un groupe alkyle, un groupe alcoxy, un groupe alkylthio, un groupe aryle, un groupe aryloxy, un groupe arylthio, un groupe aryl-alkyle, un groupe aryl-alcoxy, un groupe aryl-alkylthio, un groupe aryl-alcényle, un groupe aryl-alcynyle, un groupe hétérocyclique monovalent, un groupe hétérocyclyl-thio, un groupe amino, un groupe silyle, un groupe acyle, un groupe acyloxy, un résidu de type imine, un groupe de type amide, un groupe de type imide acide, un groupe carboxyle, un groupe cyano ou un groupe nitro.

5. Copolymère à blocs conforme à l'une des revendications 1 à 4, pour lequel, dans la formule (IV), les indices p et m valent O.

6. Copolymère à blocs conforme à l'une des revendications 1 à 5, qui présente une masse molaire moyenne en poids, en équivalent polystyrène, de 1.10³ à 1.10⁷.

7. Copolymère à blocs conforme à l'une des revendications 1 à 6, dans lequel le bloc (A) présente une masse molaire moyenne en poids, en équivalent polystyrène, de 1.10³ à 1.10⁵.

8. Copolymère à blocs conforme à l'une des revendications 1 à 7, dans lequel le rapport [A]/[B] vaut d'au moins 0,1 à au plus 10, étant entendu que [A] représente la fraction molaire du bloc (A), rapportée au total des blocs (A) et (B), et que [B] représente la fraction molaire du bloc (B), rapportée au total des blocs (A) et (B).

9. Composition **caractérisée en ce qu'**elle comprend :
un copolymère à blocs conforme à l'une des revendications 1 à 8, et un solvant, un matériau photo-émetteur autre que le copolymère à blocs, un matériau à transport de trous autre que le copolymère à blocs, ou un matériau à transport d'électrons autre que le copolymère à blocs, ou encore une combinaison de deux de ces composants ou plus.

10. Composition conforme à la revendication 9, contenant des copolymères à blocs d'au moins deux types.

11. Film mince photo-émetteur, **caractérisé en ce qu'**il comporte un copolymère à blocs conforme à l'une des revendications 1 à 8.

12. Film mince photo-émetteur, conforme à la revendication 11, qui comporte en outre un matériau photo-émetteur autre que le copolymère à blocs, un matériau à transport de trous autre que le copolymère à blocs, ou un matériau à transport d'électrons autre que le copolymère à blocs, ou encore une combinaison de deux de ces composants ou plus.

13. Dispositif photo-émetteur à polymère, **caractérisé en ce qu'**il comprend :
une anode,
une cathode,
et une couche organique, comprenant un copolymère à blocs conforme à l'une des revendications 1 à 8 et placée entre l'anode et la cathode.

14. Dispositif photo-émetteur à polymère, conforme à la revendication 13, dans lequel la couche organique comprend en outre un matériau photo-émetteur autre que le copolymère à blocs, un matériau à transport de trous autre que le copolymère à blocs, ou un matériau à transport d'électrons autre que le copolymère à blocs, ou encore une combinaison de deux de ces composants ou plus.

15. Dispositif photo-émetteur à polymère, conforme à la revendication 13 ou 14, dans laquelle la couche organique est une couche photo-émettrice.
